# EUROPEAN PATENT SPECIFICATION

(11) **EP 1 917 929 B1**
(45) Date of publication and mention of the grant of the patent: **28.04.2010**
(21) Application number: 07119953.3
(22) Date of filing: 05.11.2007
(51) Int. Cl.: A61B 19/00

(54) **Manipulator**
Manipulator
Manipulateur

(30) Priority: 06.11.2006 JP 2006300530
(43) Date of publication of application: 07.05.2008
(73) Proprietor: Terumo Kabushiki Kaisha, Tokyo 151-0072 (JP); Kabushiki Kaisha Toshiba, Minato-ku, Tokyo 105-8001 (JP)
(72) Inventor: Jinno, Makoto, Minato-ku, Tokyo 105-8001 (JP); Sunaoshi, Takamitsu, Minato-ku, Tokyo 105-8001 (JP); Uenohara, Shuichi, Fujinomiya-shi, Shizuoka 418-0015 (JP)
(74) Representative: TBK-Patent

(56) References cited:
- WO-A-99/50721
- US-A1- 2005 240 178
- US-B1- 6 394 998

## Description

### BACKGROUND OF THE INVENTION

### Field of the Invention:

The present invention relates to a manipulator according to claim 1 for actuating a working unit through power transmitting members by operating an operating unit.

### Description of the Related Art:

Heretofore, there has been used in the art a medical manipulator having an end working unit and a hand operating unit which are connected to each other by a connector. Under endoscopic observation, the hand operating unit is held by hand and operated to insert the end working unit into a body cavity and then actuate the end working unit to perform various medical treatments on the living tissue.

JP-A-2003-61969 and JP-A-2002-102248 disclose a manipulator having a working unit which incorporates a pair of grippers for gripping a living tissue. The grippers can be opened and closed about a gripper shaft, and can be angularly movable in unison about a pitch axis and a roll axis. A wire is trained around the output shaft of a motor housed in the operating unit and a pulley housed in the working unit through a connector. The torque that is required to operate the working unit is transmitted from the motor through the wire to the pulley, and then from the pulley to gears.

If forceps for use in laparoscopic surgery are used as a monopolar electrosurgical knife, then terminals disposed near the operating unit supply an electric current through electrically conductive structural members and power transmitting members to a gripper, a blade, a hook, or the like on the distal end for treating a living tissue as desired. The forceps have a shaft covered or coated with an electric insulator for preventing the electric current from flowing to the living tissue when the portion of the forceps other than the gripper or the like is brought into contact with the living tissue.

If a manipulator (multi-freedom forceps) is used as a monopolar electrosurgical knife, then as with the above forceps, terminals disposed near the operating unit supply an electric current through electrically conductive structural members and power transmitting members to a gripper or the like on the distal end for treating a living tissue as desired. The manipulator has a shaft covered or coated with an electric insulator for preventing the electric current from flowing from the shaft to the living tissue.

The manipulator has joints for making bending motions about pitch and yaw axes, and wires and gears as power transmitting members are exposed in those joints. These exposed wires and gears are possibly brought into contact with the living tissue, allowing the electric current to flow to the living tissue. Since the wires and gears disposed in the joints serve as power transmitting members, it is difficult to make them of an electrically insulating material or cover or coat them with an electric insulator.

One solution is to fully cover the joints with insulating bellows, flexible covers, hard covers, or the like. However, it is difficult to mount bellows or flexible covers on the joints which make bending motions in a relatively large angular range. Even if bellows or flexible covers are mounted on the joints, they take up a large space around the joints. If hard covers are used to cover the joints, then they need some mechanical structures to hold themselves in place, require large installation spaces, and result in very large outside diameters. Unless the covered joints are sufficiently hermetically sealed, body fluids and the blood tend to enter the joints. The bellows and covers are liable to pose obstacles to efforts to clean the joints. In other words, it is desirable that the joints have some openings for allowing the manipulator to be cleaned easily and also allowing the joints to move in large angular ranges.

WO 99/50721 A discloses a manipulator having the features according to the preamble of claim 1.

Furthermore, US 6 394 998 B1 and US 2005/240178 A1 disclose further manipulators.

### SUMMARY OF THE INVENTION

It is the object of the present invention to provide a manipulator which prevents power transmitting members such as wires, gears, etc. from contacting objects to be processed by the manipulator at joints of the manipulator.

This object is solved by a manipulator having the features of claim 1.

Advantageous further developments are subject of the dependent claims.

The above and other objects, features, and advantages of the present invention will become more apparent from the following description when taken in conjunction with the accompanying drawings in which preferred embodiments of the present invention are shown by way of illustrative example.

### BRIEF DESCRIPTION OF THE DRAWINGS

FIG. 1 is a perspective view of a manipulator according to a first embodiment of the present invention and a modification thereof;
FIG. 2 is an enlarged perspective view of a working unit of the manipulator according to the first embodiment of the present invention;
FIG. 3 is an exploded perspective view of the working unit shown in FIG. 2;
FIG. 4 is a sectional side elevational view of the working unit shown in FIG. 2;
FIG. 5 is a plan view, partly cut away, of the working unit shown in FIG. 2;
FIG. 6 is an exploded perspective view of a wire securing mechanism;
FIG. 7 is a schematic view of a drive mechanism of the manipulator according to the first embodiment of the present invention;
FIG. 8 is a cross-sectional view taken along line VIII - VIII of FIG. 4, showing the manner in which power transmitting members are prevented from contacting a living tissue by an insulating plate;
FIG. 9 is a cross-sectional view illustrative of an appropriate size for the insulating plate shown in FIG. 8;
FIG. 10 is a cross-sectional view showing a structure free of the insulating plate shown in FIG. 8;
FIG. 11 is a plan view, partly cut away, showing the manner in which the working unit shown in FIG. 5 is bent about a first rotational axis;
FIG. 12 is an enlarged perspective view of a working unit of a manipulator according to a modification of the first embodiment;
FIG. 13 is an exploded perspective view of the working unit shown in FIG. 12;
FIG. 14 is a cross-sectional side elevational view of the working unit shown in FIG. 12;
FIG. 15 is a plan view, partly cut away, of the working unit shown in FIG. 12;
FIG. 16 is a schematic view of a drive mechanism of the manipulator according to the modification of the first embodiment of the present invention;
FIG. 17 is a perspective view of a manipulator according to a third embodiment of the present invention and a modification thereof;
FIG. 18 is an enlarged perspective view of a working unit of the manipulator according to the third embodiment of the present invention;
FIG. 19 is an exploded perspective view of the working unit shown in FIG. 18;
FIG. 20 is a cross-sectional side elevational view of the working unit shown in FIG. 18;
FIG. 21 is a plan view, partly cut away, of the working unit shown in FIG. 18;
FIG. 22 is a schematic view of a drive mechanism of the manipulator according to the third embodiment of the present invention;
FIG. 23 is a cross-sectional view taken along line XXIII - XXIII of FIG. 20, showing the manner in which power transmitting members are prevented from contacting a living tissue by two insulating plates;
FIG. 24 is a perspective view of a working unit of a manipulator according to a modification of the third embodiment;
FIG. 25 is an exploded perspective view of the working unit shown in FIG. 24;
FIG. 26 is a perspective view of a modified insulating plate;
FIG. 27 is a perspective view of another modified insulating plate;
FIG. 28 is a plan view, partly cut away, showing the manner in which a working unit incorporating the insulating plate shown in FIG. 26 is bent about a first rotational axis; and
FIG. 29 is a plan view, partly cut away, showing the manner in which a working unit incorporating the insulating plate shown in FIG. 27 is bent about a first rotational axis.

### DESCRIPTION OF THE PREFERRED EMBODIMENTS

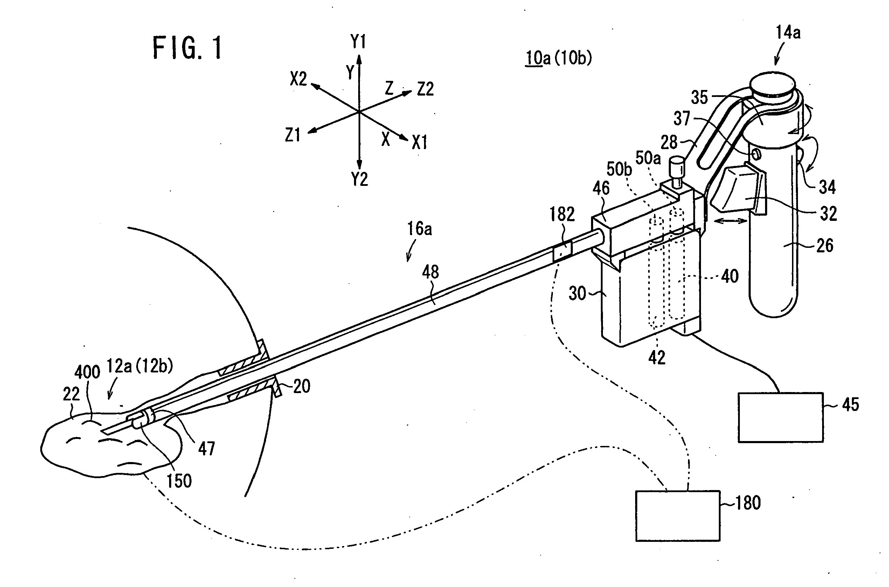

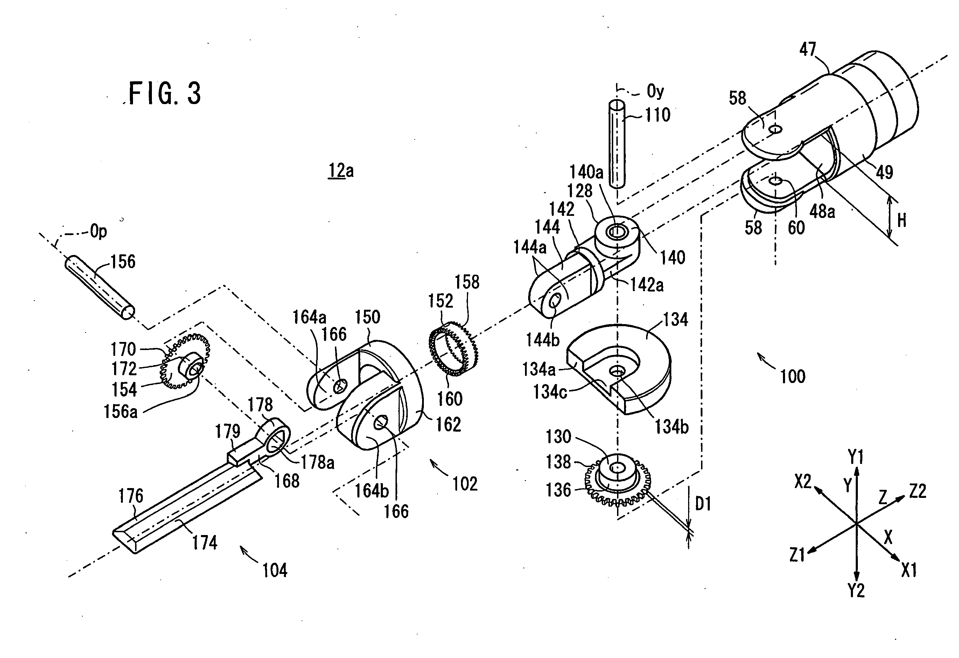

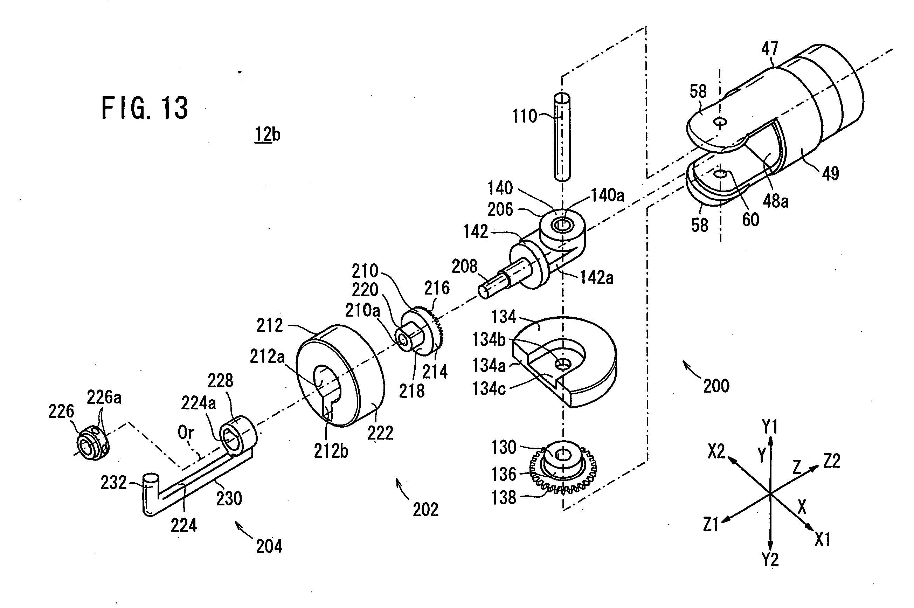

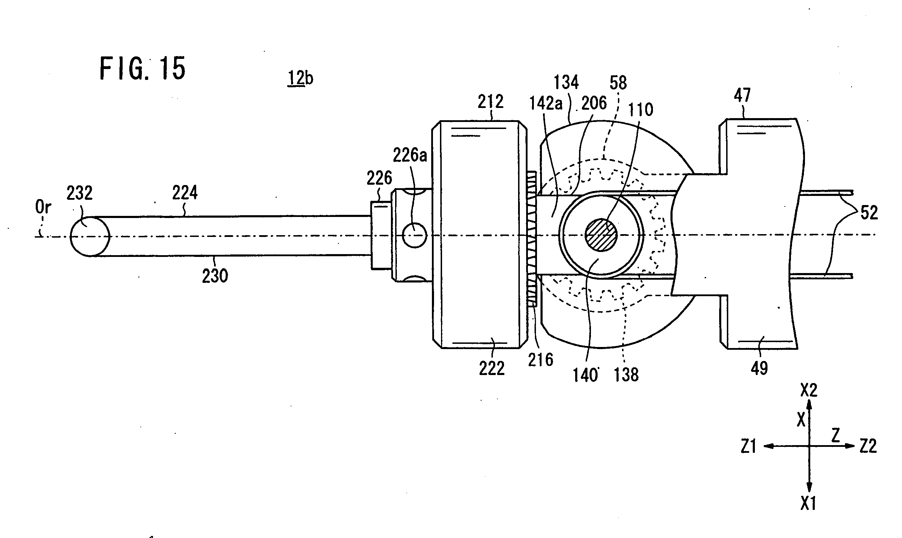

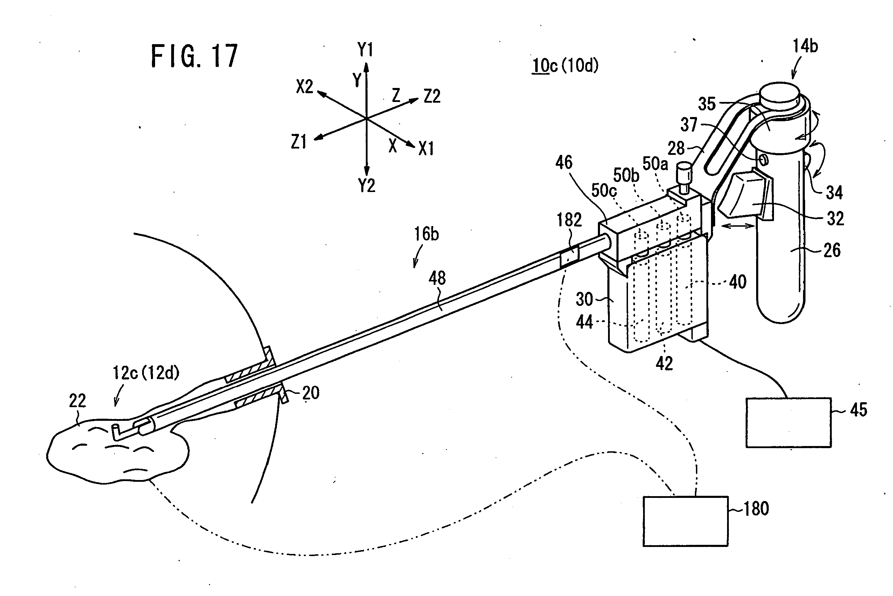

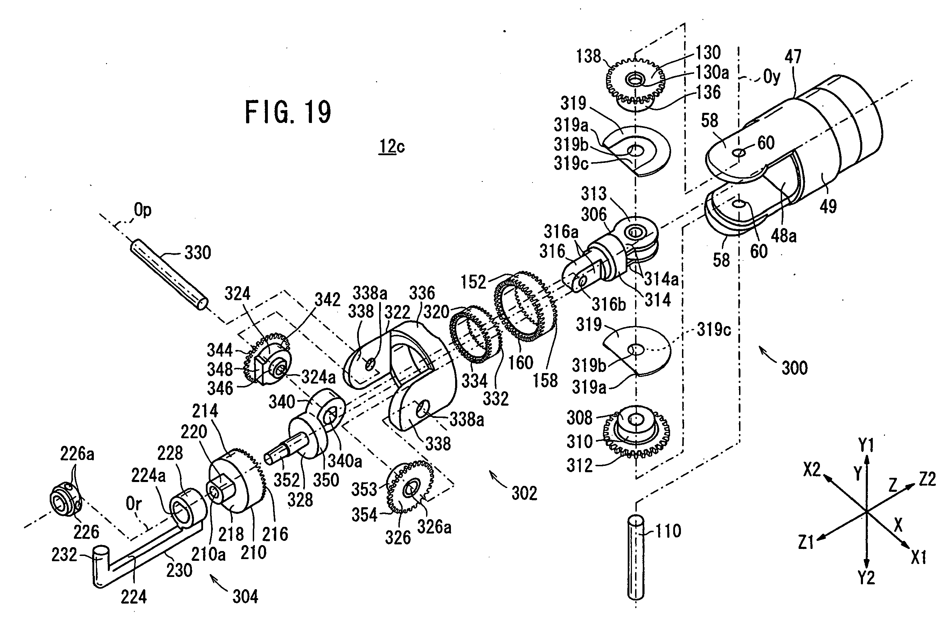

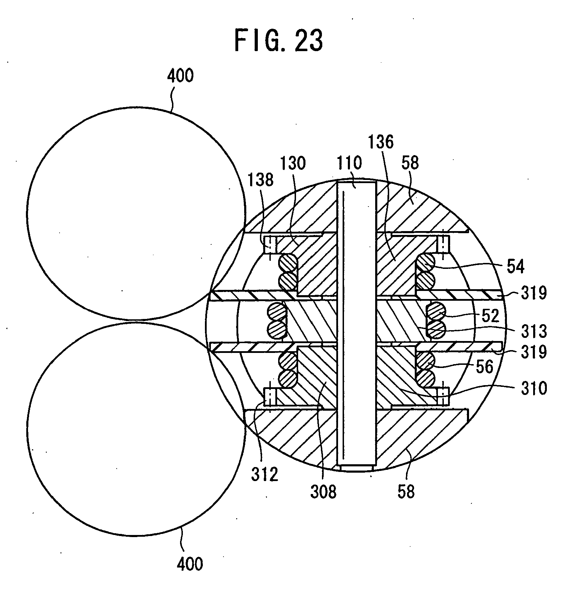

Manipulators (multi-freedom forceps) according to first and second embodiments and their modifications of the present invention will be described below with reference to FIGS. 1 through 29. A manipulator 10a according to a first embodiment and a manipulator 10b according to a modification thereof (see FIG. 1), a manipulator 10c according to a second embodiment and a manipulator 10d according to a modification thereof (see FIG. 17), are in the form of medical manipulators for use in laparoscopic surgical operations or the like. Working units 12a, 12b, 12c, 12d according to the embodiments comprise mechanisms having two or three degrees of freedom and mounted on the distal ends of the manipulators 10a, 10b, 10c, 10d.

As shown in FIG. 1, the manipulator 10a functions as a monopolar electrosurgical knife for passing an electric current from the working unit 12a at the distal end to a living tissue to heat a desired area of the living tissue. The manipulator 10a comprises an operation command unit (operating unit) 14a on a proximal end thereof which is held and operated by hand, the working unit 12a on the distal end thereof for working on a living tissue, and an elongate connector 16a interconnecting the working unit 12a and the operation command unit 14a. The working unit 12a and the connector 16a are of a small diameter and can be inserted into a body cavity 22 through a small hole (trocar) 20 defined in an abdominal region or the like of the patient. The working unit 12a is actuated by the operation command unit 14a to perform a desired process (heating process) on a desired region in the body cavity 22.

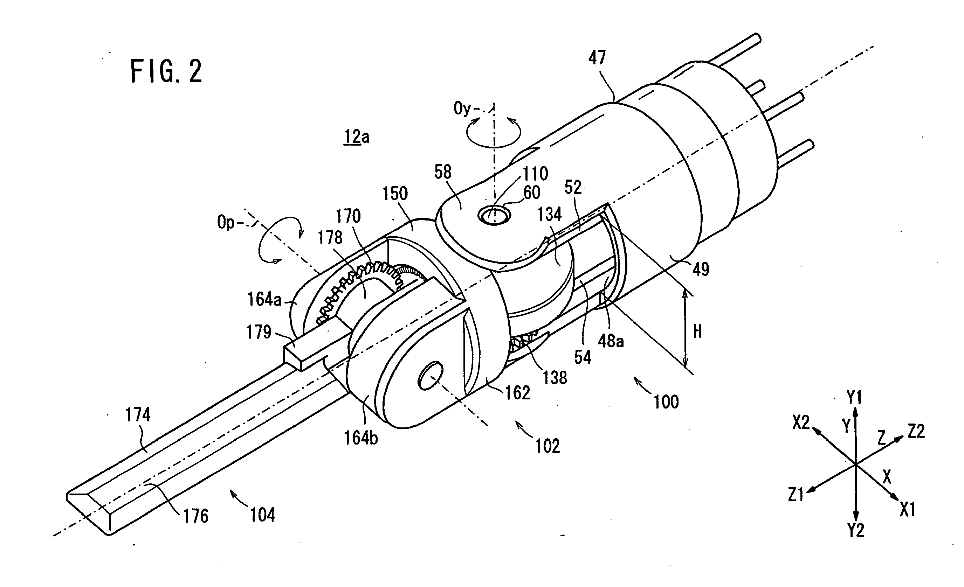

It is assumed in the description which follows that transverse directions of each of the manipulators 10a through 10d are referred to as X direction, vertical directions as Y direction, and longitudinal directions of a connector 16a and a connector 16b (see FIG. 17) as Z direction in FIGS. 1, 12, 17, and 24. Of the X direction, the rightward direction is referred to as an X1 direction, and the leftward direction as an X2 direction. Of the Y direction, the upward direction is referred to as a Y1 direction, and the downward direction as a Y2 direction. Of the Z direction, the forward direction is referred to as a Z1 direction, and the rearward direction as a Z2 direction. Unless otherwise noted, these directions represent directions of the manipulators 10a through 10d when they are of a neutral posture (shown in FIGS. 2, 12, 18, and 24). The definition of the above directions is for illustrative purpose only, and the manipulators 10a through 10d can be used in any orientations, e.g., it may be used upside down.

The operation command unit 14a includes a grip handle 26 gripped by hand, an arm 28 extending from an upper portion of the grip handle 26, and an actuator block (actuator) 30 connected to a distal end of the arm 28. The grip handle 26 includes a trigger lever 32 which can be operated by a finger, a vertical roller 34, a horizontal roller 35, and a switch 37. The trigger lever 32 is disposed in a position where it can easily be pulled by the index finger. The vertical roller 34 and the horizontal roller 35 are disposed in respective positions where they can easily be rotated by the thumb.

The actuator block 30 houses therein motors 40, 42 corresponding to respective mechanisms of two degrees of freedom which are incorporated in the working unit 12a. The motors 40, 42 are arrayed parallel to each other in the longitudinal direction of the connector 16a. The motors 40, 42 are small in size and diameter, making the actuator block 30 compact and flat in shape. The actuator block 30 is disposed underneath the end of the connector 16a in the Z2 direction. The motors 40, 42 can be energized to rotate their drive shafts under the master-slave control of a controller 45 based on commands (operation) of the operation command unit 14a (master).

The connector 16a includes a proximal joint 46 joined to the actuator block 30 and a hollow connector shaft 48 extending in the Z1 direction from the proximal joint 46. The proximal joint 46 houses therein a drive pulley (first rotational source) 50a and a drive pulley (second rotational source) 50b which are rotatable that are connected respectively to the drive shafts of the motors 40, 42. A wire (first flexible power transmitting member) 52 and a wire (second flexible power transmitting member) 54 are trained respectively around the drive pulleys 50a, 50b and extend through a hollow region 48a (see FIG. 2) in the connector shaft 48 to the working unit 12a. The wires 52, 54 may be of the same type and same diameter.

The connector 16a can be detached from the operation command unit 14a with a predetermined process on the proximal joint 46 for cleaning, sterilization, maintenance, etc. The connector 16a and the working unit 12a can be replaced with other connectors and working units. For example, depending on the technique required for a certain surgical operation, the connector 16a (connector shaft 48) may be replaced with a connector having a different length and/or the working unit 12a may be replaced with a working unit incorporating a different mechanism. If the actuator block 30 is integrally combined with the operation command unit 14a, then the proximal joint 46 of the connector 16a may be connected to the operation command unit 14a (the arm 28).

As shown in FIG. 2, the connector 16a has a distal joint 47 disposed on the distal end thereof. The distal joint 47 serves to connect the connector 16a and the working unit 12a to each other and also to protect and support various parts of a drive mechanism 102, to be described later, of the working unit 12a. The distal joint 47 has a short sleeve 49 extending in the Z2 direction and a pair of diametrically opposite tongues 58 projecting from respective upper and lower ends of the short sleeve 49 toward the distal end thereof in the Z1 direction, the tongues 58 being disposed in parallel in facing relation to the central axis of the connecting shaft 48. The hollow region 48a in the connector shaft 48 communicates with a space between the tongues 58. The tongues 58 have respective shaft holes 60 defined respectively therein which are held in alignment with each other. The tongues 58 have respective distal ends which are arcuate in shape. The tongues 58 have respective flat inner surfaces facing each other which extend parallel to each other and which are spaced from each other by a distance H.

The two shaft holes 60 are disposed in nearly symmetrical positions one on each side of the central axis of the connector 16a. The shaft holes 60 are positioned substantially at the centers of the arcuate distal ends of the tongues 58.

The working unit 12a incorporates therein a mechanism of two degrees of freedom. The mechanism includes a first degree of freedom for angularly moving a portion of the working unit 12a that is positioned ahead of a first rotational axis Oy extending along the Y direction, in yawing directions about the first rotational axis Oy, and a second degree of freedom for angularly moving the portion of the working unit 12a in pitching directions about a second rotational axis Op extending along the X direction. The working unit 12a comprises a wire-driven mechanism 100, a drive mechanism 102, and an end effector (acting unit) 104. Though the drive mechanism 102 and the end effector 104 will hereinafter be described separately from each other as a matter of convenience, since the term "end effector" is generally interpreted as a mechanism on an arm end for performing a certain action, the end effector 104 and the drive mechanism 102 may be defined as an integrated end effector.

The wire-driven mechanism 100, the drive mechanism 102, and the end effector 104 will be described in detail below with reference to FIGS. 2 through 5.

The wire-driven mechanism 100 is disposed between the tongues 58 and serves to convert circulative movements of the respective wires 52, 54 into rotational movements and transmit the rotational movements to the drive mechanism 102. The wire-driven mechanism 100 includes a shaft (first joint shaft) 110 inserted in the shaft holes 60, a main shaft assembly 128 rotatably supported on the shaft 110, and a gear body 130. The shaft 110 is press-fitted securely in the shaft holes 60, and is axially aligned with the first rotational axis Oy.

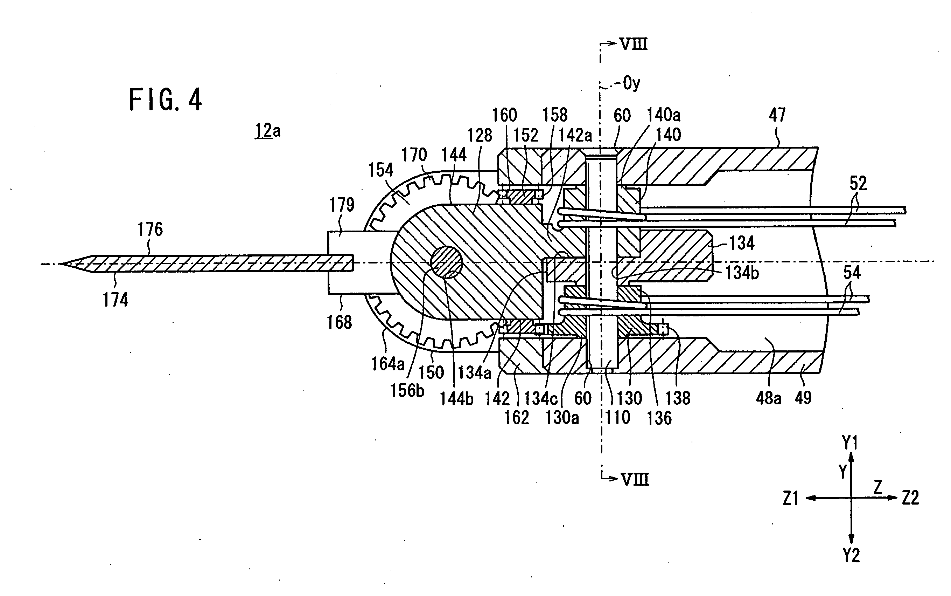

The gear body 130 comprises a tubular member (second tubular member) 136 and a first gear 138 disposed concentrically on a lower portion of the tubular member 136. The first gear 138 comprises a spur gear greater in diameter than the tubular member 136. Unless otherwise specified, a gear referred to herein comprises a spur gear. The first gear 138 has a thickness D1 which is sufficiently smaller than the distance H. The first gear 138 has a low annular rib 130a disposed on the lower surface thereof around the hole through which the shaft 110 is inserted. The annular rib 130a prevents the lower surface of the first gear 138 from contacting the lower tongue 58, thereby reducing the sliding resistance that is imposed on the first gear 138 by the lower tongue 58 (see FIG. 4).

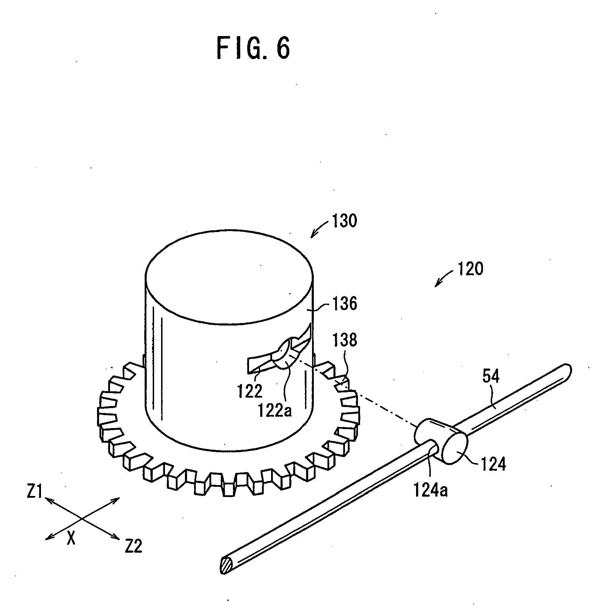

As shown in FIG. 6, the tubular member 136 is combined with a wire securing mechanism 120. The wire securing mechanism 120 has a groove 122 defined in a substantially central portion of the side of the tubular member 136 which faces the Z2 direction and extending laterally in the X direction when the gear body 130 is in a neutral position, and a tapered fastening pin 124 disposed centrally in the groove 122. The groove 122 has a hole 122a positioned centrally for the fastening pin 124 to be inserted and fixed therein. The groove 122 may be slightly skewed in alignment with a turn of the wire 54 that is helically wound around the tubular member 136.

The groove 122 has a width and a maximum depth that are essentially equal to the diameter of the wire 54. The fastening pin 124 has a hole 124a defined laterally therethrough for the wire 54 to extend therethrough. The wire 54 is threaded through the hole 122a and the fastening pin 124 is inserted into the hole 124a, holding the wire 54 partly in the groove 122. The wire 54 is thus oriented horizontally and fastened to the tubular member 136.

As shown in FIGS. 2 through 5, the main shaft assembly 128 has a tubular member (first tubular member) 140 through which the shaft 110 extends, an annular seat 142 coupled to the tubular member 140 and facing in the Z1 direction, and a pitch base 144 extending from the center of the annular seat 142 in the Z1 direction. The pitch base 144 is a member serving as a basis for movement in the pitching directions, and includes a pair of laterally spaced parallel slide surfaces 144a for defining movement in the pitching directions and a hole 144b defined in the distal end thereof and extending between the slide surfaces 144a. The annular seat 142 is slightly spaced from the tubular member 140 with a bridge 142a interposed therebetween, the bridge 142a extending in the Z1 direction from a region slightly lower than the center of an outer circumferential surface of the tubular member 140. The tubular member 140 has on its upper surface a low annular rib 140a extending around the hole through which the shaft 110 is inserted. The annular rib 140a prevents the upper surface of the tubular member 140 from contacting the upper tongue 58, thereby reducing the sliding resistance that is imposed on the tubular member 140 by the upper tongue 58 (see FIG. 4). The tubular member 140 is combined with a wire securing mechanism 120, which is similar to the wire securing mechanism 120 of the tubular member 136, on the side of the tubular member 140 which faces in the Z2 direction, and the wire 52 is fastened to the tubular member 140 by the wire securing mechanism 120.

In response to circulative movement of the wire 52, the main shaft assembly 128 rotates in the yawing directions about the first rotational axis Oy to cause the pitch base 144 to swing in an XZ plane.

The wire-driven mechanism 100 also includes an insulating plate (first insulating member) 134 rotatably supported on the shaft 110 between the tubular member 140 and the tubular member 136.

The insulating plate 134 is in the form of a partly circular plate made of PEEK (polyetheretherketone), for example, having a flat side surface 134a. The insulating plate 134 has a hole 134b defined therein substantially at the center of the arcuate shape thereof for the shaft 110 to be inserted therethrough. The insulating plate 134 includes a counterbore 134c defined in an upper surface thereof for receiving the tubular member 140 and the bridge 142a engaging therein. The counterbore 134c has a depth substantially equal to the thickness of the bridge 142a.

The tubular member 140, the insulating plate 134, and the gear body 130 are stacked together along the shaft 110 and have a combined height which is essentially equal to the distance H such that they are disposed with substantially no clearances between the tongues 58 (see FIG. 4).

The drive mechanism 102 comprises a cover 150, a gear ring 152 and a gear body 154 which are housed in the cover 150, and a securing pin (second joint shaft) 156 on which the gear body 154 is rotatably supported.

The gear ring 152 is in the form of a thin tubular member including a face gear 158 on an end face thereof facing in the Z2 direction and a face gear 160 on an end face thereof facing in the Z1 direction. The gear ring 152 is fitted over the annular seat 142 of the main shaft assembly 128 for sliding rotation with respect to the outer circumferential surface of the annular seat 142 (see FIG. 4). The face gear 158 is in mesh with the first gear 138, so that the gear ring 152 is rotatable about the central axis of the connector 16a in response to rotation of the gear body 130.

The cover 150 serves to protect and support the components of the drive mechanism 102. The cover 150 includes a short tube 162 extending in the Z2 direction, an ear 164a projecting in the Z1 direction from an end of the short tube 162 in the X2 direction, and an ear 164b projecting in the X2 direction from a region of the short tube 162 which is located inwardly in the Z1 direction from an opposite end of the short tube 162 in the X1 direction, the ears 164a, 164b facing each other. The ear 164b is thicker than the ear 164a. The ears 164a, 164b have respective holes 166 defined therein for inserting the securing pin 156 therein. The securing pin 156 is press-fitted and secured in the holes 166, for example. The ears 164a, 164b have respective parallel surfaces confronting each other, and are spaced from each other by such a distance that the gear body 154, an engaging member 168, and the pitch base 144 are slidably held between the ears 164a, 164b. The short tube 162 has an inner circumferential surface whose diameter is slightly greater than the diameter of the outer circumferential surface of the gear ring 152, with a clearance left therebetween (see FIG. 4). The engaging member 168 serves as one of the components of the end effector 104.

The gear body 154 is positioned in a region between the ears 164a, 164b which is displaced in the X2 direction from the center of the cover 150, and includes a second gear 170 and a boss 172 coupled centrally to the second gear 170 in concentric alignment therewith on one side thereof in the X1 direction and having a D-shaped cross section. The second gear 170 is held in mesh with the face gear 160. The gear body 154 has a central hole 156a defined therein through which the securing pin 156 is inserted.

The end effector 104 comprises an end effector member 174 having a blade (electrode unit) 176 and the engaging member 168.

The blade 176 is in the form of an elongate thin plate. The engaging member 168 which is transversely offset in the X2 direction projects in the Z2 direction from a side portion of the proximal end of the blade 176.

The engaging member 168 comprises a short tube 178 having a central axis aligned with the second rotational axis Op and a bridge 179 interconnecting the short tube 178 and the blade 176. The short tube 178 has a hole 178a of a D-shaped cross section defined centrally therein for receiving the boss 172 snugly therein.

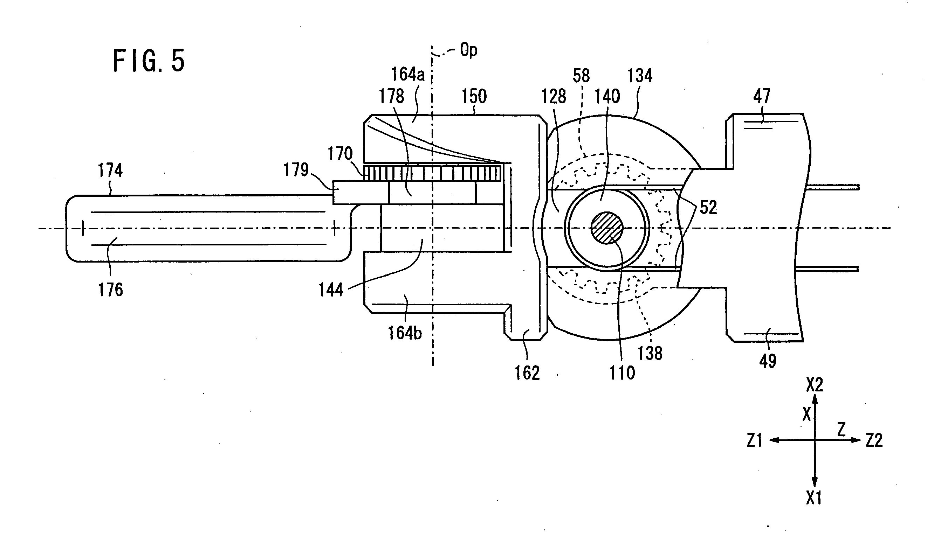

The gear body 154, the engaging member 168, and the pitch base 144 are stacked together along the securing pin 156 such that they are disposed with substantially no clearances between the ears 164a, 164b. The securing pin 156 is inserted and supported in the holes 156a, 144b (see FIG. 5). When the gear ring 152 rotates about its own axis, the end effector member 174 is swingable about the second rotational axis Op. At this time, the gear body 154 is rotated by the gear ring 152.

Specifically, in the working unit 12a, the rotation of the gear body 130 is transmitted from the first gear 138 through the gear ring 152 to the gear body 154, angularly lifting or lowering the blade 176 about the second rotational axis Op.

The wire 52 is wound as 1.5 turns or more around the tubular member 140 of the main shaft assembly 128, and the wire 54 as 1.5 turns around the tubular member 136 of the gear body 130 (see FIG. 4).

Operation of the manipulator 10a thus constructed will be described below. Since there are mechanical interferences in the drive mechanism, the operation of the drive pulleys (the drive motors) and the operation of the rotational axes (posture axes) do not held in 1-to-1 correspondence to each other. However, for the sake of brevity, the operation of the drive pulleys (the drive motors) and the operation of the rotational axes (posture axes) will be described below as being held in 1-to-1 correspondence to each other.

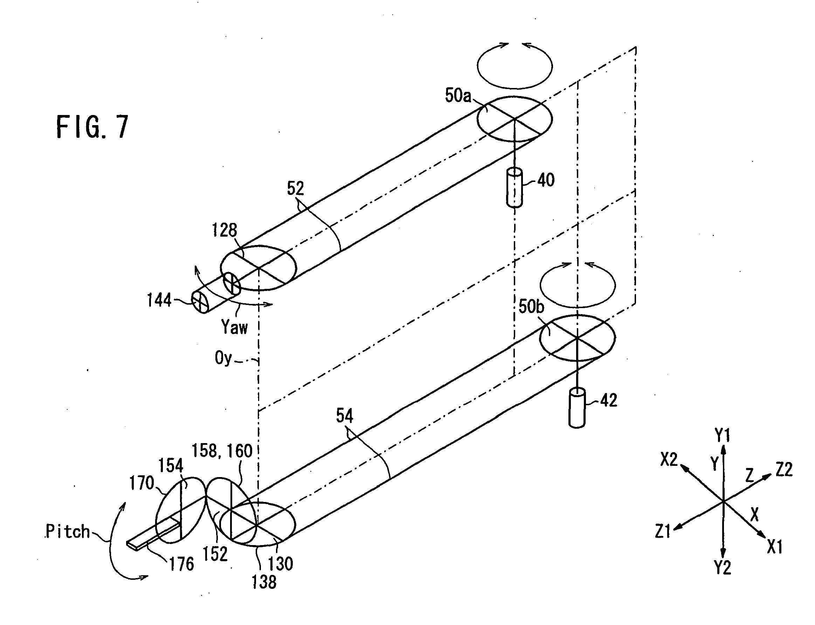

The switch 37 of the operation command unit 14a is turned on to activate the manipulator 10a. Then, as shown in FIG. 7, the end effector 104 is actuated in a yawing direction by operating the horizontal roller (first input unit) 35 (see FIG. 1) with a finger. Specifically, when the operator rotates the horizontal roller 35 to the left or right through a certain angle with a finger, the motor 40 is energized to rotate the drive pulley 50a to circulatively move the wire 52, rotating the main shaft assembly 128 about the first rotational axis Oy. The drive mechanism 102 and the end effector 104 that are connected to the pitch base 144 of the main shaft assembly 128 are now caused to swing in the yawing direction. At this time, since the bridge 142a of the main shaft assembly 128 engages in the counterbore 134c of the insulating plate 134, the insulating plate 134 also rotates in response to the rotation of the main shaft assembly 128.

When the horizontal roller 35 is rotated to the left or right (in one or reverse direction), the end effector 104 is also turned to the left or right (in one or reverse direction) depending on the direction in which the horizontal roller 35 is rotated. When the horizontal roller 35 is stopped at a certain angle, the motor 40 is de-energized, stopping the end effector 104 against further movement in the yawing direction and holding it in the stopped position.

The end effector 104 is actuated in a pitching direction by operating the vertical roller (second input unit) 34 (see FIG. 1) with a finger. Specifically, when the operator rotates the vertical roller 34 upwardly or downwardly through a certain angle with a finger, the motor 42 is energized to rotate the drive pulley 50b to circulatively move the wire 54, rotating the gear body 130 about the first rotational axis Oy. The rotation is transmitted from the gear body 130 through the first gear 138, the face gears 158, 160, and the second gear 170 to the gear body 154. The gear body 154 is turned upwardly or downwardly about the second rotational shaft Op in unison with the engaging member 168 coupled thereto by the boss 172. The blade 176 is thus turned upwardly or downwardly about the second rotational shaft Op.

When the vertical roller 34 is rotated upwardly or downwardly (in one or reverse direction), the end effector 104 is also turned upwardly or downwardly in response to the rotation of the vertical roller 34, i.e., is also turned in the Y1 direction or the Y2 direction depending on the direction in which the vertical roller 34 is rotated. When the vertical roller 34 is stopped at a certain angle, the motor 42 is de-energized, stopping the end effector 104 against further movement in the pitching direction and holding it in the stopped position.

The manipulator 10a is typically used as a monopolar electrosurgical knife. When the manipulator 10a operates as a monopolar electrosurgical knife, a power supply 180 supplies an electric current from a terminal 182 (see FIG. 1) to the blade 176 through various structural members and power transmitting members which include the connector 16a, the distal joint 47, the gear bodies 130, 154, the main shaft assembly 128, the wires 52, 54, the gear ring 152, the engaging member 168, the shaft 110, and the securing pin 156. The blade 176 is thus heated to treat a living tissue 400 (see FIG. 1) of a human body. Specifically, the electric current from the power supply 180 flows through a power supply cable and the terminal 182 into the manipulator 10a, and then flows from the end effector 104 into the human body to be treated. The electric current then returns from an electrode plate held against the human body through a power supply cable to the power supply 180. Since the blade 176 can be angularly moved freely in the yawing and pitching directions, as described above, the blade 176 can easily be manipulated to supply the electric current to a desired region of the human body to treat the region.

The connector 16a, the distal joint 47, and the cover 150 which have outer circumferential surfaces that may contact the living tissue 400 (see FIG. 1) are not power transmitting members. Therefore, the connector 16a, the distal joint 47, and the cover 150 may be made of an insulating material or may be coated with an insulating material. If the connector 16a, the distal joint 47 and the cover 150 are made of or coated with an insulating material, then even when they are brought into contact with the living tissue 400, no electric current flows from them into the living tissue 400.

On the other hand, the gear body 130, the main shaft assembly 128, and the wires 52, 54, etc. must not be made of or coated with an insulating material as they are power transmitting members. If all these power transmitting members are made of an insulating material, then it is impossible to supply a desired electric current from the power supply 180 to the blade 176.

In the working unit 12a, the insulating plate 134 is effective to prevent the gear bodies 130, the main shaft assembly 128, and the wires 52, 54, etc. from being exposed out of the joint which has the first rotational shaft Oy at its center.

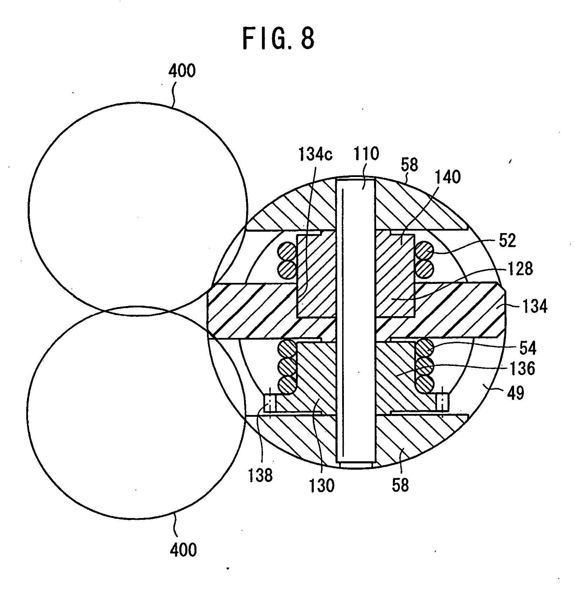

Specifically, as shown in FIGS. 5 and 8, if the radius of the arcuate shape of the insulating plate 134 is sufficiently large, e.g., as large as the radius of the short sleeve 49, then the first gear 138, the tubular members 136, 140, and the wires 52, 54 are prevented from being exposed out of the joint. Thus, the first gear 138, etc. and the living tissue 400 remain spaced apart a sufficient distance from each other, and are prevented from contacting each other. In other words, the insulating plate 134 serves as a shield for protecting the living tissue 400 from being contacted by the first gear 138, etc., and functions as a shield member for preventing an accidental current flow from the first gear 138, etc. to the living tissue 400. Stated otherwise, the insulating plate 134 functions as a spacer for keeping the living tissue 400 and the first gear 138, etc. spaced apart from each other.

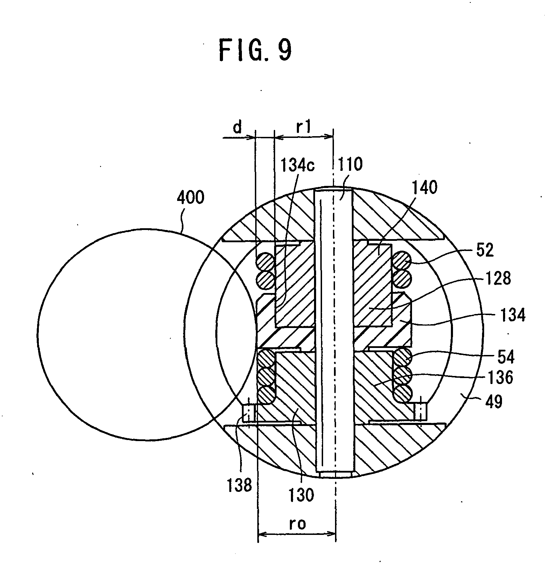

As shown in FIG. 9, if the radius (r0) of the arcuate shape (circular plate) of the insulating plate 134 is equal to or greater than the sum of the radius (r1) of the tubular member 140 and the diameter (d) of the wire 52 or the sum of the radius of the tubular member 136 and the diameter of the wire 54, then the outer circumferential surface of the insulating plate 134 is reliably positioned outwardly of the tubular members 140, 136 and the wires 52, 54. Accordingly, the living tissue 400 and the first gear 138, etc. remain spaced from each other by an appropriate distance.

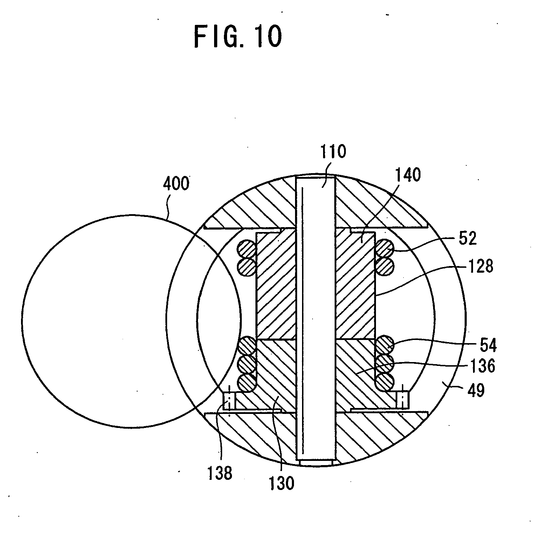

If the wire-driven mechanism is free of the insulating plate 134 as with the related art (see FIG. 10), then since the first gear 138, the tubular members 136, 140, and the wires 52, 54 are exposed in the joint, they tend to contact the living tissue 400. In other words, it is highly likely for the living tissue 400 to enter the joint from between the tongues 58 and contact the first gear 138, etc.

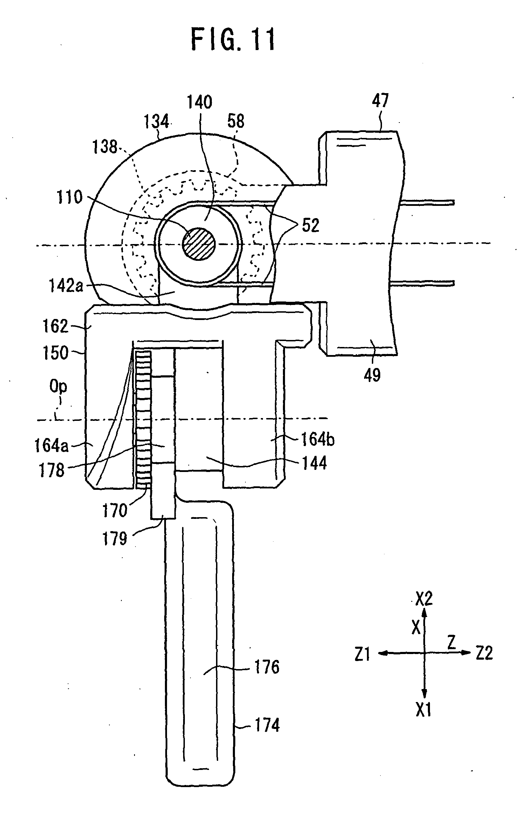

Furthermore, as shown in FIG. 11, even when the drive mechanism 102 and the end effector 104 are operated in a yawing direction substantially through a right angle to expose the first gear 138, etc. from the joint through an increased range about the first rotational axis Oy, the opening of the joint is effectively closed by the insulating plate 134. In particular, as the insulating plate 134 of the arcuate shape is rotatable with the main shaft assembly 128 in the working unit 12a, the insulating plate 134 turns in unison with the drive mechanism 102, etc. even when the joint is largely bent. Therefore, the arcuate shape (circular plate) of the insulating plate 134 can keep the living tissue 400 and the first gear 138, etc. spaced from each other by a constant distance. In the working unit 12a, therefore, since the insulating plate 134 turns in response to the swinging movement in yawing directions about the first rotational axis Oy while the end effector 104 can be moved in a sufficient range and can be cleaned effectively, the insulating plate 134 is continuously capable of preventing the living tissue 400 and the first gear 138, etc. from contacting each other.

The insulating plate 134 should preferably be made of an insulating (electrically nonconductive) material for the purpose of preventing the first gear 138, etc. and the living tissue 400 from accidentally contacting each other. Specifically, the insulating plate 134 should preferably be made of a synthetic resin material such as PEEK or the like as described above or a ceramics material such as zirconia or the like. If the insulating plate 134 is to be sterilized at high temperatures, then it needs to withstand a sufficiently high thermal deformation temperature of 132°C or higher, for example. In addition, as the working unit 12a is used within the human body, the insulating plate 134 should be of a material which is free of brittle fracture and does not diffusely reflect light from an endoscope.

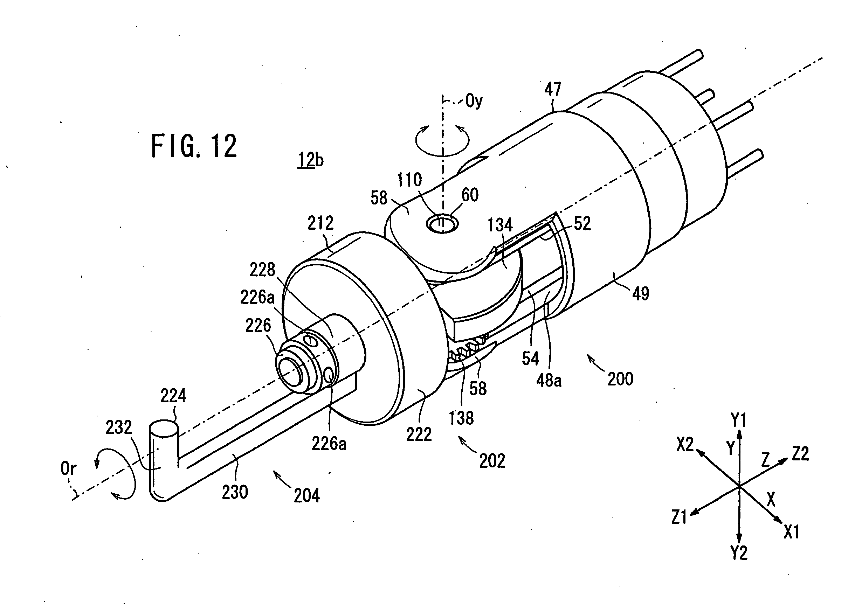

A manipulator 10b according to a modification of the manipulator 10a according to the first embodiment will be described below with reference to FIGS. 12 through 16.

The manipulator 10b is similar to the manipulator 10a in that it has the same operation command unit 14a and the same connector 16a, but is different from the manipulator 10a in that it has a working unit 12b instead of the working unit 12a. The working unit 12b comprises a wire-driven mechanism 200, a drive mechanism 202, and an end effector (acting unit) 204.

As shown in FIG. 12, the working unit 12b incorporates therein a mechanism of two degrees of freedom. The mechanism includes a first degree of freedom for angularly moving a portion of the working unit 12b that is positioned ahead of the first rotational axis Oy extending along the Y direction, in yawing directions about the first rotational axis Oy, and a second degree of freedom for angularly moving the portion of the working unit 12b in rolling directions about the second rotational axis Or extending along the Z direction.

The wire-driven mechanism 200 corresponds to the wire-driven mechanism 100, and includes a main shaft assembly 206 instead of the main shaft assembly 128 of the wire-driven mechanism 100. The main shaft assembly 206 is different from the main shaft assembly 128 in that it includes a main shaft (second joint shaft) 208 in place of the pitch base 144. The main shaft 208 projects in the Z1 direction from the annular seat 142.

In the wire-driven mechanism 200, the wires 52, 54 are wound around the tubular member 140 and the tubular member 136, as with the wire-driven mechanism 100. In the wire-driven mechanism 200, the main shaft assembly 206 (the main shaft 208) can be turned in yawing directions about the first rotational axis Oy.

The drive mechanism 202 has a crown 210 and a cover 212.

The crown 210 includes a thin tubular member 214, a face gear 216 disposed on a surface of the tubular member 214 which faces in the Z2 direction, a disk 218 closing the end of the tubular member 214 which faces in the Z1 direction, and a boss 220 projecting in the Z1 direction from the center of the disk 218 and having a D-shaped cross section. The disk 218 and the boss 220 have a hole 210a defined therein for inserting the main shaft 208 therein. The face gear 216 is held in mesh with the first gear 138.

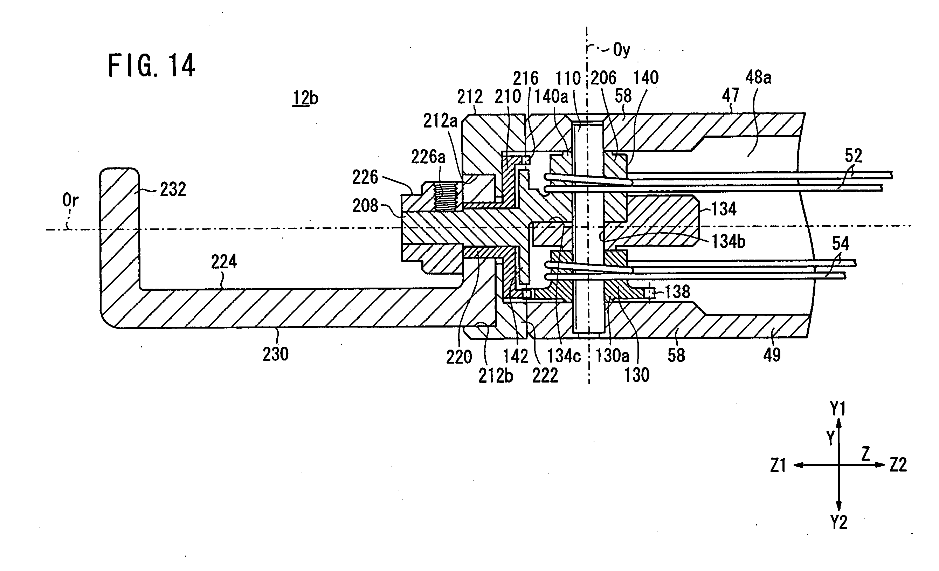

The cover 212 has an outside diameter which is substantially the same as the outside diameter of the distal joint 47. The cover 212 has a tubular member 222 in which the tubular member 214 is inserted, a hole 212a in which the boss 220 is inserted, and a rectangular hole 212b extending in the Y2 direction from the hole 212a. The tubular member 222 has an inner circumferential surface slightly larger in diameter than the outer circumferential surface of the crown 210, with a clearance left therebetween (see FIG. 14). The cover 212 is made of or coated with an insulating material, as with the cover 150.

The end effector 204 comprises a hook (electrode unit) 224 and a fastening nut 226.

The hook 224 comprises a coupling 228 connected to the drive mechanism 202, an arm 230 extending in the Z1 direction from the coupling 228 at a position slightly offset from the second rotational axis Or, and an electrode 232 bent from the distal end of the arm 230 in the Y1 direction. The coupling 228 has a hole 224a of a D-shaped cross section into which the boss 220 of the crown 210 is inserted.

When the main shaft 208 is inserted into the crown 210, the cover 212, and the hook 224, the fastening nut 226 secures them together. The fastening nut 226 has a plurality of radial small holes 226a defined therein for inserting a narrow rotary tool. When the coupling 228 and the arm 230 of the hook 224 are inserted into the hole 212a and the rectangular hole 212b of the cover 212, the boss 220 into the hole 224a, and the main shaft 208 into the hole 210a of the crown 210, the main shaft 208 has its distal end projecting in the Z1 direction from the coupling 228. The fastening nut 226 is threaded over the projecting end of the main shaft 208, thus assembling the end effector 204 on the drive mechanism 202. The crown 210, the cover 212, and the hook 224 are now rotatably supported on the main shaft 208.

In the end effector 204, the crown 210 and the hook 224 are rotatable about the second rotational axis Or in response to rotation of the gear body 130 and the first gear 138.

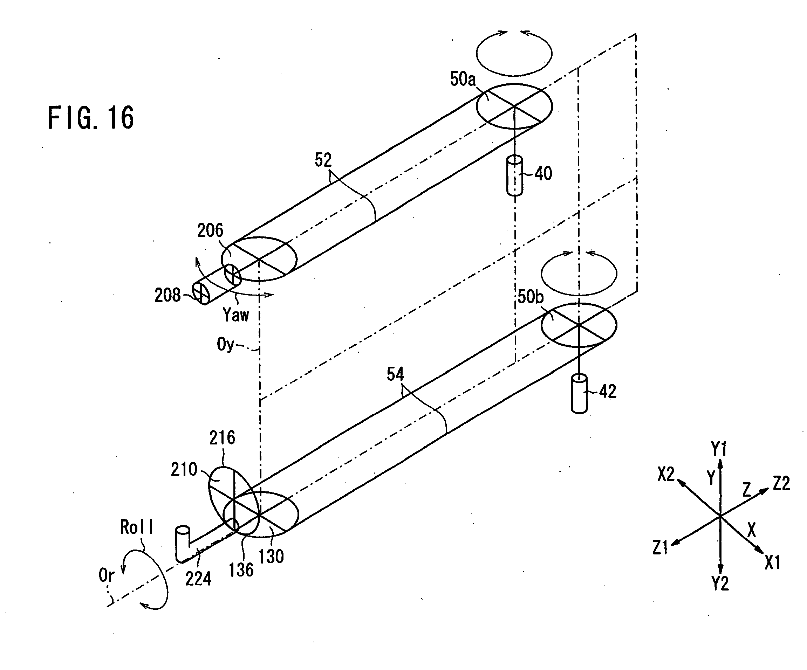

Operation of the manipulator 10b thus constructed will be described below with reference to FIG. 16.

The switch 37 of the operation command unit 14a is turned on to activate the manipulator 10b. Then, as shown in FIG. 16, the end effector 204 is actuated in a yawing direction by operating the horizontal roller 35 (see FIG. 1) with a finger. Specifically, when the operator rotates the horizontal roller 35 to the left or right through a certain angle with a finger, the motor 40 is energized to rotate the drive pulley 50a to circulatively move the wire 52, rotating the main shaft assembly 206 about the first rotational axis Oy. The drive mechanism 202 and the end effector 204 that are connected to the main shaft 208 of the main shaft assembly 206 are now caused to swing in the yawing direction. At this time, as with the working unit 12a, since the bridge 142a of the main shaft assembly 206 engages in the counterbore 134c of the insulating plate 134, the insulating plate 134 also rotates in response to the rotation of the main shaft assembly 206.

The end effector 204 is actuated in a rolling direction by turning the vertical roller 34 (see FIG. 1) with a finger. Specifically, when the operator rotates the vertical roller 34 upwardly or downwardly through a certain angle with a finger, the motor 42 is energized to rotate the drive pulley 50b to circulatively move the wire 54, rotating the gear body 130 about the first rotational axis Oy. The rotation is transmitted from the gear body 130 through the first gear 138 to the face gear 216. The hook 224 of the end effector 204 is rotated about the second rotational shaft Op in unison with the boss 220 of the crown 210.

As with the manipulator 10a, the manipulator 10b can be used to treat a living tissue. Specifically, the power supply 180 supplies an electric current from the terminal 182 (see FIG. 1) to the hook 224 through various structural members and power transmitting members which include the connector 16a, the distal joint 47, the gear body 130, the main shaft assembly 206, the wires 52, 54, the crown 210, the coupling 228, and the shaft 110. The hook 224 is thus heated to treat the living tissue 400 of the human body. As the hook 224 can be angularly moved freely in the yawing and rolling directions, as described above, the hook 224 can easily be manipulated to supply the electric current to a desired region of the human body to treat the region.

The manipulator 10b has the insulating plate 134 as with the manipulator 10a. Accordingly, the living tissue 400 is effectively prevented from contacting the gear body 130, the main shaft assembly 206, and the wires 52, 54 in the joint with the first rotational axis Oy at its center. In particular, even when the end effector 204 is largely bent in a yawing direction, e.g., substantially through a right angle, about the first rotational axis Oy, the insulating plate 134 turns in unison with the main shaft assembly 206 to close the opening of the joint. Therefore, while the end effector 204 can be moved in a sufficient range and can be cleaned effectively, the insulating plate 134 is continuously capable of preventing the gear body 130, etc. and the living tissue 400 from contacting each other.

With the manipulator 10b, the outer circumferential surface of the crown 210 is protected by the cover 212 which is made of or coated with an insulating material, as described above. Therefore, the crown 210 is also prevented from contacting the living tissue 400.

A manipulator 10c according to a second embodiment of the present invention will be described below with reference to FIGS. 17 through 21.

The manipulator 10c is different from the manipulator 10a in that it has a connector 16b and an operation command unit (operating unit) 14b instead of the connector 16a and the operation command unit 14a, and it has a working unit 12c instead of the working unit 12a.

The operation command unit 14b includes, in the actuator block 30, a motor 44 in addition to the motors 40, 42, disposed parallel to the motors 40, 42, for operating respective mechanisms having three degrees of freedom provided in the working unit 12c.

The proximal joint 46 of the connector 16b houses therein a drive pulley (third rotational source) 50c connected to the drive shaft of the motor 44. A wire (third flexible power transmitting member) 56 is trained around the drive pulley 50c and extends through the hollow region 48a in the connector shaft 48 to the working unit 12c, as with the wires 52, 54. The wire 56 is of the same type and same diameter as the wires 52, 54. The connector 16b can be detached from the operation command unit 41b, in the same way as in the connector 16a.

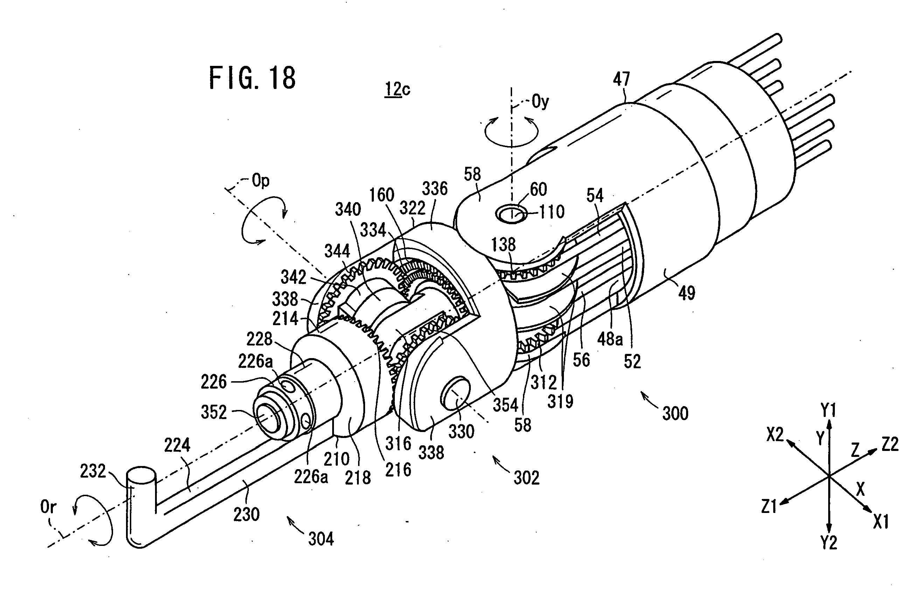

The working unit 12c comprises a wire-driven mechanism 300, a drive mechanism 302, and an end effector (acting unit) 304. As shown in FIG. 18, the working unit 12c incorporates therein a mechanism of three degrees of freedom. The mechanism includes a first degree of freedom for angularly moving a portion of the working unit 12c that is positioned ahead of a first rotational axis Oy extending along the Y direction, in yawing directions about the first rotational axis Oy, a second degree of freedom for angularly moving the end effector 304 in pitching directions about a second rotational axis Op extending along the X direction, and a third degree of freedom for angularly moving the end effector 304 about a third rotational axis Or extending along the Y direction.

The wire-driven mechanism 300 corresponds to the wire-driven mechanism 100, and comprises a gear body 130, a main shaft assembly 306, and a gear body 308 which are rotatably supported on the shaft 110 and arranged successively in the order named in the Y2 direction. The shaft 110 has an externally threaded distal end which is threaded and secured in one of the shaft holes 60 which is internally threaded in the Y1 direction.

The gear body 130 is essentially identical in shape to the gear body 130 of the wire-driven mechanism 100, but is in an upside-down orientation with respect thereto about the central axis of the connector shaft 48. Stated otherwise, the first gear 138 is mounted on the upper end of the tubular member 136.

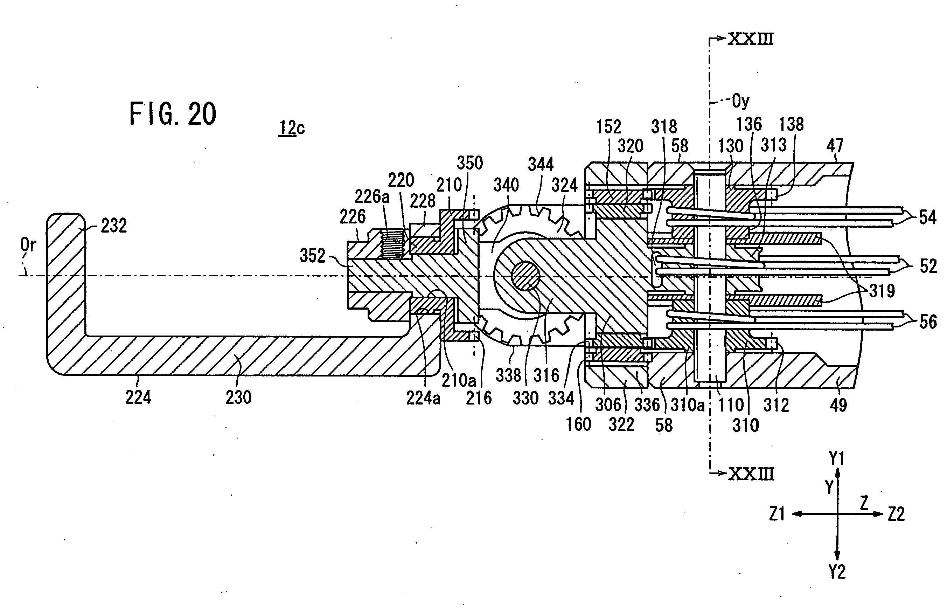

The gear body 308 comprises a tubular member (third tubular member) 310 and a third gear 312 disposed concentrically on a lower portion of the tubular member 310. The third gear 312 has a low annular rib 310a disposed on the lower surface thereof around the hole through which the shaft 110 is inserted. The annular rib 310a prevents the lower surface of the third gear 312 from contacting the lower tongue 58, thereby reducing the sliding resistance that is imposed on the third gear 312 by the lower tongue 58 (see FIG. 20). The tubular member 310 is combined with a wire securing mechanism 120, which is the same as the wire securing mechanism 120 on the tubular member 136, on the side of the tubular member 310 which faces in the Z2 direction, and the wire 56 is fastened to the tubular member 310 by the wire securing mechanism 120.

The main shaft assembly 306 disposed between the gear bodies 130, 308 comprises a tubular member 313 through which the shaft 110 extends, a slightly thick annular seat 314, and a pitch base 316 extending from the center of the annular seat 314 in the Z1 direction. The pitch base 316 is a member serving as a basis for movement in the pitching directions, as with the pitch base 144, and includes a pair of laterally spaced parallel slide surfaces 316a for defining movement in the pitching directions and a hole 316b defined in a distal end thereof to provide a rotational center.

The annular seat 314 is slightly spaced from the outer circumferential surface of the tubular member 313 with two upper and lower short bridges 314a interposed therebetween. A vertical hole 318 which is slightly elongate in the Y direction is defined between the annular seat 314 and the tubular member 313 for receiving the wire 52 to extend therethrough (see FIG. 20). The tubular member 313 is combined with a wire securing mechanism 120, which is similar to the wire securing mechanism 120 of the tubular member 140, on the side of the tubular member 313 which faces in the Z2 direction, and the wire 52 is fastened to the tubular member 313 by the wire securing mechanism 120.

In the wire-driven mechanism 300, the wires 52, 54, 56 are wound respectively around the tubular members 313, 136, 310. The wire-driven mechanism 300 allows the main shaft assembly 306 to swing in the yawing directions about the first rotational axis Oy.

The wire-driven mechanism 300 also has two insulating plates (a first insulating member and a second insulating member) 319 which are rotatably supported respectively between the tubular member 136 and the tubular member 313 and between the tubular member 313 and the tubular member 310.

Each of the insulating plates 319, which corresponds to the insulating plate 134, is in the form of a partly circular plate thinner than the insulating plate 134 and having a flat side surface 319a. The insulating plate 319 has a hole 319b defined therein substantially at the center of the arcuate shape thereof for the shaft 110 to be inserted therethrough. One of the insulating plates 319 which is offset from the other insulating plate 319 in the Y1 direction includes a counterbore 319c defined in an upper surface thereof for receiving the tubular member 136 engaging therein. Similarly, the other insulating plate 319 that is offset in the Y2 direction includes a counterbore 319c defined in a lower surface thereof for receiving the tubular member 310 engaging therein. These counterbores 319c have a depth substantially one-half of the thickness of the insulating plate 319.

The radius of the arcuate shape (circular plate) of each of the insulating plates 319 should preferably be equal to or greater than the sum of the radius of the tubular member 136 and the diameter of the wire 54, or the sum of the radius of the tubular member 313 and the diameter of the wire 52, or the sum of the radius of the tubular member 310 and the diameter of the wire 56. Particularly, if the radius of the arcuate shape (circular plate) of each of the insulating plates 319 is equal to or greater than the greatest value among the sum of the radius of the tubular member 136 and the diameter of the wire 52, the sum of the radius of the tubular member 313 and the diameter of the wire 54, and the sum of the radius of the tubular member 310 and the diameter of the wire 56, then the outer circumferential surface of the insulating plate 319 is reliably positioned outwardly of the tubular members 136, 313, 310 and the wires 52, 54, 56. Accordingly, the living tissue 400 and the first gear 138, etc. remain spaced from each other by an appropriate distance. The insulating plates 319 may be made of the same material as the insulating plate 134.

With the wire-driven mechanism 300, the tubular member 313 is disposed substantially centrally between the tongues 58, and the tubular members 136, 310 are disposed respectively above and below the tubular member 313. The two thin insulating plates 319 are effective to prevent the power transmitting members including the main shaft assembly 306, the first gear 138, the third gear 312, and the wires 52, 54, 56, etc. from being exposed out of the joint.

The drive mechanism 302 comprises a cover 322, a gear ring 152, a gear ring 320, a gear body 324, and a gear body 326 which are housed in the cover 322, an end effector main shaft 328, and a securing pin (second joint shaft) 330 on which the gear rings and the gear bodies are rotatably supported.

The gear ring 320 is in the form of a thin tubular member including a face gear 332 on an end face thereof facing in the Z2 direction and a face gear 334 on an end face thereof facing in the Z1 direction. The gear ring 320 is fitted in the gear ring 152 for sliding rotation with respect to the inner circumferential surface of the gear ring 152. The face gear 332 is held in mesh with the third gear 312, so that the gear ring 320 is rotatable about the third rotational axis Or in response to rotation of the gear body 308.

The cover 322, which corresponds to the cover 150, serves to protect and support the components of the drive mechanism 302. The cover 322 includes a short tube 336 extending in the Z2 direction and a pair of ears 338 projecting in the Z1 direction from respective opposite side portions of the short tube 336. The ears 338 have respective holes 338a defined therein for inserting and securing the securing pin 330 therein. The securing pin 330 is press-fitted and secured in the holes 338a, for example. The ears 338 have respective parallel surfaces confronting each other, and have such a width that the gear bodies 324, 326, an engaging member 340 of the end effector main shaft 328, and the pitch base 316 are slidably held by the ears 338. The short tube 336 has an inner circumferential surface whose diameter is slightly greater than the diameter of the outer circumferential surface of the gear ring 152, with a clearance left therebetween (see FIG. 20). As with the cover 150, the cover 322 is made of or coated with an insulating material.

The gear body 324 is positioned in a region between the ears 338 which is displaced in the X2 direction from the center of the cover 322, and includes a tubular member 342, a fourth gear 344 mounted concentrically on one end of the tubular member 342, a boss 346 having a D-shaped cross section mounted concentrically on the other end of the tubular member 342, and a support base 348 disposed on a side of the tubular member 342 which faces in the Z1 direction. The gear body 324 is oriented such that the fourth gear 344 faces in the X2 direction. The fourth gear 344 is held in mesh with the face gear 160. The gear body 324 has a central hole 324a defined therein through which the securing pin 330 is inserted.

The end effector main shaft 328 comprises a base disk 350, a main shaft (third joint shaft) 352 projecting in the Z1 direction from the base disk 350, and an engaging member 340 projecting in the Z2 direction from a surface of the base disk 350 which faces in the Z2 direction at a position that is slightly displaced in the X2 direction from the center of the base disk 350. The engaging member 340 has a hole 340a of a D-shaped cross section in which the boss 346 engages. When the boss 346 is inserted into the hole 340a, the end effector main shaft 328 is integrally and stably combined with the gear body 324 with the base disk 350 and the support base 348 being held in face-to-face contact with each other.

The gear body 326 is positioned in a region between the ears 338 which is displaced in the X1 direction from the center of the cover 322, and includes a tubular member 353 and a fifth gear 354 coupled to an end surface of the tubular member 353 in concentric alignment therewith. The gear body 326 is oriented such that the fifth gear 354 faces in the X1 direction. The fifth gear 354 is held in mesh with the face gear 334 of the gear ring 320. The gear body 326 has a central hole 326a defined therein through which the securing pin 330 is inserted.

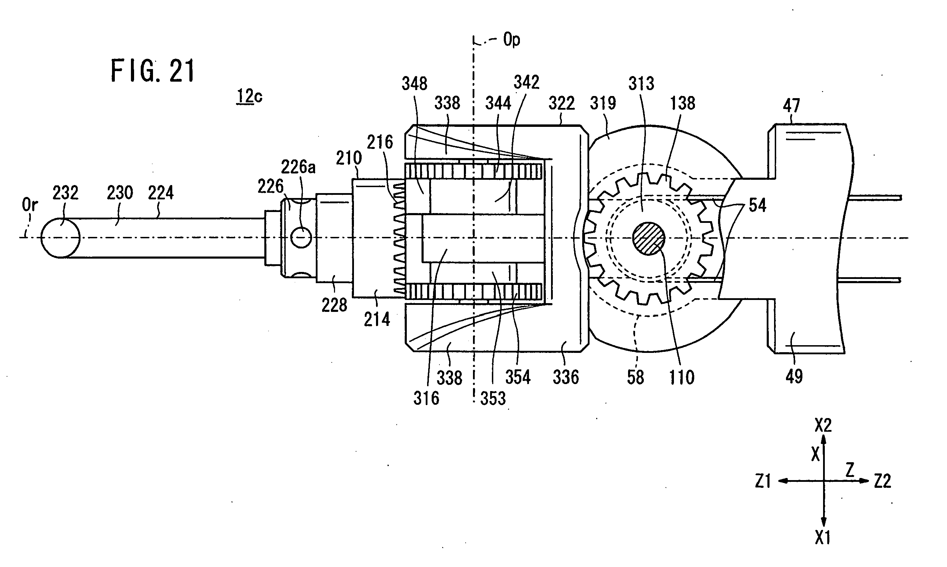

The assembly of the gear body 326, the end effector main shaft 328, the pitch base 316, and the gear body 324 is disposed with substantially no clearances between the ears 338. The securing pin 330 is inserted through the holes 324a, 316b, 326a and supported therein (see FIG. 21). The assembly of the end effector main shaft 328 and the gear body 324 is swingable about the second rotational axis Op in response to rotation of the gear ring 152. The gear body 326 is rotatable in response to rotation of the gear ring 320.

In the drive mechanism 302 thus constructed, the rotation of the gear body 130 and the first gear 138 is transmitted through the gear ring 152 and the fourth gear 344 to the main shaft 352, which is angularly lifted or lowered about the second rotational axis Op. The rotation of the gear body 308 and the third gear 312 is transmitted through the gear ring 320 to the gear body 326 and the fifth gear 354.

The end effector 304 comprises a crown 210, a hook (electrode unit) 224, and a fastening nut 226.

When the boss 220 is inserted into the hole 224a and the main shaft 352 of the drive mechanism 302 into the hole 210a of the crown 210, the main shaft 352 has its distal end projecting in the Z1 direction from the coupling 228. The fastening nut 226 is threaded over the projecting end of the main shaft 352, thus assembling the end effector 304 on the drive mechanism 302. The crown 210 and the hook 224 are now rotatably supported on the main shaft 352. The face gear 216 is held in mesh with the fifth gear 354. In the end effector 304, the crown 210 and the hook 224 is rotatable about the third rotational axis Or in response to rotation of the gear body 326 and the fifth gear 354.

Operation of the manipulator 10c thus constructed will be described below.

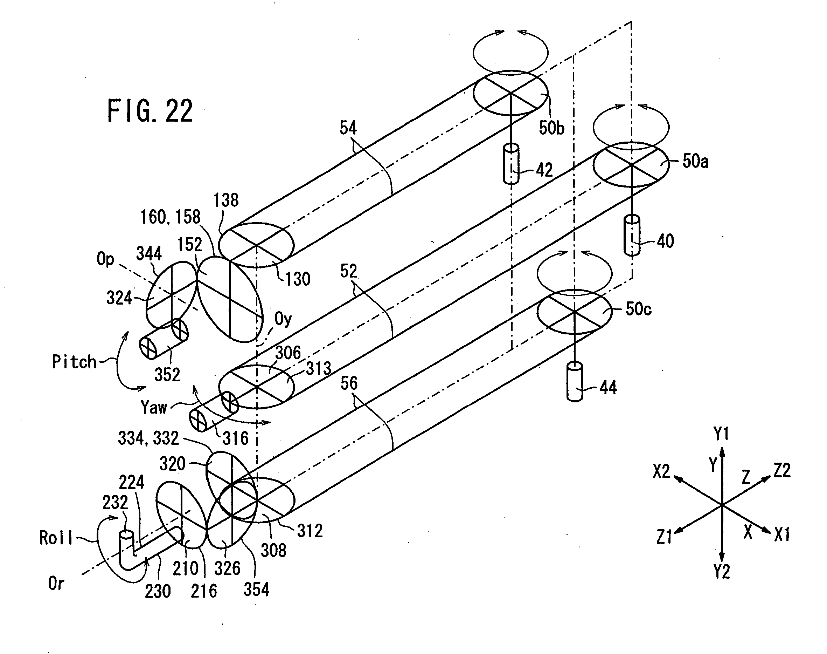

The switch 37 of the operation command unit 14b is turned on to activate the manipulator 10c. Then, as shown in FIG. 22, the end effector 304 is actuated in a yawing direction by operating the horizontal roller 35 (see FIG. 1) with a finger. Specifically, when the operator rotates the horizontal roller 35 to the left or right with a finger, the motor 40 is energized to rotate the drive pulley 50a to circulatively move the wire 52, rotating the main shaft assembly 306 about the first rotational axis Oy. The drive mechanism 302 and the end effector 404 that are connected to the pitch base 316 of the main shaft assembly 306 are now caused to swing in the yawing direction. At this time, as with the working unit 12a, the two insulating plates 319 also rotate in response to the rotation of the main shaft assembly 306.

The end effector 304 is actuated in a pitching direction by operating the vertical roller 34 (see FIG. 1) with a finger. Specifically, when the operator rotates the vertical roller 34 upwardly or downwardly through a certain angle with a finger, the motor 42 is energized to rotate the drive pulley 50b to circulatively move the wire 54, rotating the gear body 130 about the first rotational axis Oy. The rotation is transmitted from the gear body 130 through the first gear 138, the face gears 158, 160, and the fourth gear 344 to the gear body 324. The gear body 324 is turned upwardly or downwardly about the second rotational shaft Op in unison with the engaging member 340 coupled thereto by the boss 346. The hook 224 is thus turned upwardly or downwardly about the second rotational shaft Op by the engaging member 340 through the main shaft 352.

The end effector 304 is actuated in a rolling direction by pulling the trigger lever (third input unit) 32 (see FIG. 1) with a finger. Specifically, when the operator pulls the trigger lever 32 with a finger, the motor 44 is energized to rotate the drive pulley 50c to circulatively move the wire 56, rotating the gear body 308, whose rotation is transmitted through the third gear 312, the face gears 332, 334, and the fifth gear 354 to the face gear 216. The hook 224 of the end effector 304 is rotated in unison with the boss 220 of the crown 210 about the third rotational axis Or.

The trigger lever 32 can be pulled by a finger, and return to its original position under resiliency when it is released from the finger. The end effector 304 operates in ganged relation to the trigger lever 32 such that the end effector 304 is rotated in the rolling direction depending on how much the trigger lever 32 is pulled. When the trigger lever 32 returns to its original position, the end effector 304 also returns to its original position in the rolling direction.

With the manipulator 10c including the mechanisms having three degrees of freedom, the trigger lever 32 may function as a switch. If the trigger lever 32 functions as a switch, then when the trigger lever 32 is operated, the vertical roller 34 for operating the end effector 304 in pitching directions may selectively be used as an operating means for operating the end effector 304 in rolling directions. The posture axes at the frontal end, such as the end effector 304, of the mechanism having three degrees of freedom may be operated using other operating devices that are separately provided.

In the manipulator 10c, the power supply 180 supplies an electric current from the terminal 182 to the hook 224 through various structural members and power transmitting members which include the connector 16b, the distal joint 47, the gear bodies 130, 308, 324, 326, the main shaft assembly 306, the wires 52, 54, 56, the gear rings 152, 320, the end effector main shaft 328, the crown 210, the coupling 228, the shaft 110, and the securing pin 330. The hook 224 is thus heated to treat the living tissue 400 of the human body. As the hook 224 can be angularly moved freely in the yawing, pitching, and rolling directions, as described above, the hook 224 can easily be manipulated to supply the electric current to a desired region of the human body to treat the region.

Since the manipulator 10c has the two insulating plates 319 which correspond to the insulating plate 134 of the manipulator 10a, the insulating plates 319 prevent the living tissue 400 from contacting the main shaft assembly 306 and the wires 52, 54, 56 in the joint with the first rotational axis Oy at its center. In particular, even when the end effector 304 is largely bent in a yawing direction, e.g., substantially through a right angle, about the first rotational axis Oy, the two insulating plates 319 turn in unison with the main shaft assembly 306 to close the opening of the joint. Therefore, while the end effector 304 can be moved in a sufficient range and can be cleaned effectively, the insulating plates 319 are continuously capable of preventing the gear body 130, etc. and the living tissue 400 from contacting each other.

In the wire-driven mechanism 300 of the manipulator 10c, the tubular member 313 is disposed substantially centrally between the tongues 58 and the tubular members 136, 310 are disposed above and below the tubular member 313. Since the two thin insulating plates 319 are employed, the insulating plates 319 can be evenly disposed in the opening of the joint to effectively prevent the power transmitting members including the first gear 138, the third gear 312, the wires 52, 54, 56, etc. from being exposed out of the joint and contacting the living tissue 400.

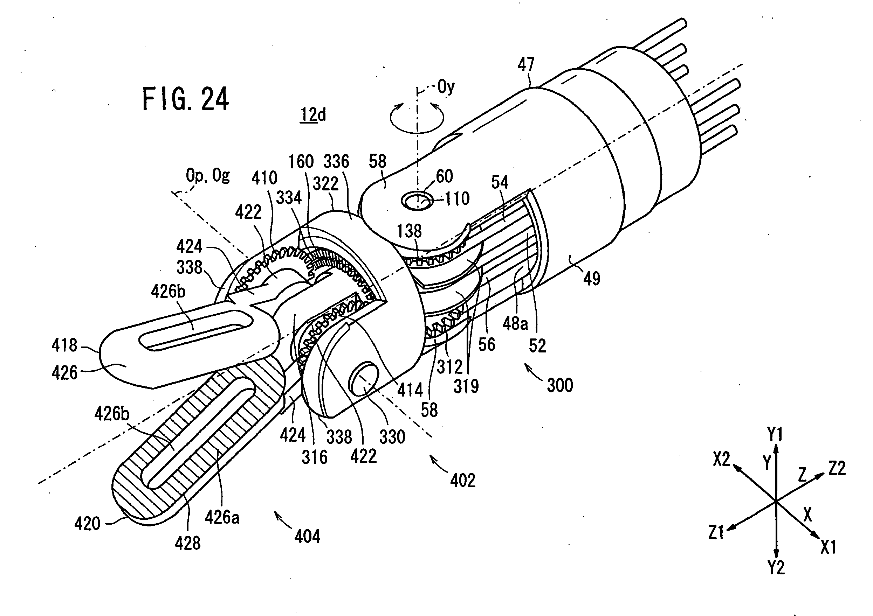

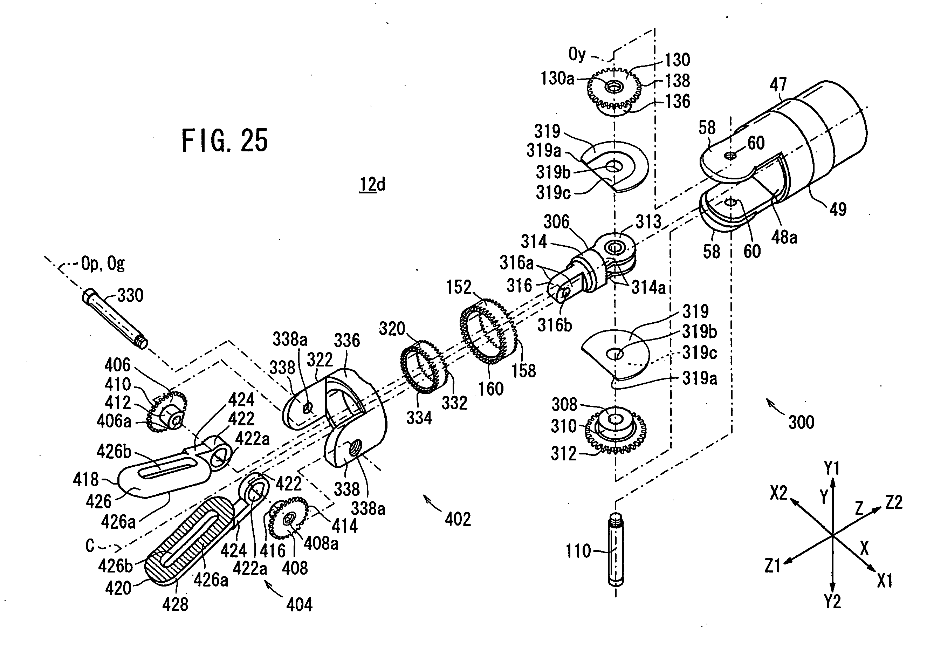

A manipulator 10d according to a modification of the manipulator 10c according to the second embodiment will be described below with reference to FIGS. 24 and 25.

The manipulator 10d is similar to the manipulator 10c in that it has the same operation command unit 14b and the same connector 16b, but is different from the manipulator 10c in that it has a working unit 12d instead of the working unit 12c. The working unit 12d comprises a wire-driven mechanism 300, a drive mechanism 402, and an end effector (acting unit) 404.

As shown in FIG. 24, the working unit 12d incorporates therein a mechanism of three degrees of freedom. The mechanism includes a first degree of freedom for angularly moving a portion of the working unit 12d that is positioned ahead of a first rotational axis (first joint axis) Oy extending along the Y direction, in yawing directions about the first rotational axis Oy, a second degree of freedom for angularly moving the end effector 404 in pitching directions about a second rotational axis (second joint axis) Op extending along the X direction, and a third degree of freedom for opening and closing the end effector 404 about a third rotational axis (third joint axis, gripper axis) Og that is coaxial with the second rotational axis Op.

The drive mechanism 402 comprises a cover 322, gear rings 152, 320, a gear body 406, and a gear body 408 which are housed in the cover 322, and a securing pin 330 on which these gear rings and gear bodies are rotatably supported.

The gear body 406 is disposed in a region between the ears 338 which is displaced in the X2 direction from the center of the cover 322, and comprises a sixth gear 410 and a boss 412 having a D-shaped cross section mounted concentrically on an end of the sixth gear 410 which faces in the X1 direction. The sixth gear 410 is held in mesh with the face gear 160. The gear body 406 has a central hole 406a defined therein through which the securing pin 330 is inserted.

The gear body 408 is disposed in a region between the ears 338 which is displaced in the X1 direction from the center of the cover 322, and comprises a seventh gear 414 and a boss 416 having a D-shaped cross section mounted concentrically on an end of the seventh gear 414 which faces in the X2 direction. The seventh gear 414 is held in mesh with the face gear 334. The gear body 408 has a central hole 408a defined therein through which the securing pin 330 is inserted.

The end effector 404 comprises a first end effector member 418 and a second end effector member 420.

The first end effector member 418 comprises a proximal end tube 422, an arm 424 projecting substantially radially in the Z1 direction from the proximal end tube 422, and a gripper 426 projecting radially in the Z1 direction from the arm 424. The proximal end tube 422 has a hole 422a of a D-shaped cross section defined centrally therein for receiving the boss 412 snugly therein. Therefore, the hole 422a serves to position the boss 412 and prevent the boss 412 from rotating about its own axis.

The gripper 426 is slightly thicker than the proximal end tube 422 and the arm 424 in the X1 direction, and has an intermediate transverse region lying substantially flush with the end surfaces of the proximal end tube 422 and the arm 424 which face in the X1 direction. The gripper 426 has opposite ends which are arcuate in shape, and includes parallel ridges disposed on an inner side surface 426a and extending in the X direction. The ridges serve to prevent a tool or the like gripped by the gripper 426 from slipping. The gripper 426 has a rectangular hole 426b defined therein along its longitudinal axis for making the gripper 426 lightweight and gripping the tool or like effectively.

The second end effector member 420 is identical in shape to the first end effector member 418, and has a gripper 428 which is identical in shape to the gripper 426. The second end effector member 420 is held in engagement with the boss 416 of the gear body 408, and is in an upside-down orientation with respect to the first end effector member 418. The second end effector member 420 comprise components identical to those of the first end effector member 418. Those components of the second end effector member 420 which are identical to those of the first end effector member 418 are denoted by identical reference characters, and will not be described in detail below.

The gripper 426 of the first end effector member 418 is displaced in the X1 direction from the gripper 428 of the second end effector member 420, and the gripper 428 of the second end effector member 420 is displaced in the X2 direction from the gripper 426 of the first end effector member 418. The grippers 426, 428 are disposed symmetrically with respect to the central axis of the connector shaft 48 (reference axis C in FIG. 25) such that their inner side surfaces 426a face each other.

The sixth gear 410, the seventh gear 414, the two proximal end tubes, and the pitch base 316 are stacked together along the securing pin 330 such that they are disposed with substantially no clearances between the ears 338 (see FIG. 24). The securing pin 330 is inserted and supported in the holes 406a, 316b, 408a.

When rotation of the gear ring 152 is transmitted to the sixth gear 410, the first end effector member 418 is angularly moved about the second rotational axis Op (the third rotational axis Og). When rotation of the gear ring 320 is transmitted to the seventh gear 414, the second end effector member 420 is angularly moved about the second rotational axis Op (the third rotational axis Og).

Specifically, when the gear rings 152, 320 rotate clockwise as viewed in front elevation (from the Z1 direction toward the Z2 direction along the reference axis C), the sixth gear 410 rotates counterclockwise as viewed from a side of the reference axis C, and the seventh gear 414 rotates counterclockwise as viewed from a side of the reference axis C. The arms 424 and the grippers 426, 428 are moved toward each other, i.e., closed. Conversely, when the gear rings 152, 320 rotate counterclockwise as viewed in front elevation, the sixth gear 410 rotates clockwise as viewed from a side of the reference axis C, and the seventh gear 414 rotates clockwise as viewed from a side of the reference axis C. The arms 424 and the grippers 426, 428 are moved away from each other, i.e., opened.

When the vertical roller 34 and the trigger lever 32 are operated, the gear bodies 130, 308 are rotated, and their rotation is transmitted through the gear rings 152, 320 to the sixth gear 410 and the seventh gear 414, thereby opening and closing the grippers 426, 428 about the third rotational axis Og (gripper axis).

When the vertical roller 34 and the trigger lever 32 are operated to rotate the gear rings 152, 320 in opposite directions, the grippers 426, 428 are angularly moved in the same direction, i.e., lifted or lowered in a pitching direction about the second rotational axis Op. The vertical roller 34 and the trigger lever 32 may be operated individually to lift and lower the grippers 426, 428 individually in pitching directions. The end effector 404 can be operated in yawing directions in the same manner as with the working unit 12c. Therefore, operation of the end effector 404 in yawing directions will not be described in detail below.

In the manipulator 10d, the power supply 180 supplies an electric current from the terminal 182 (see FIG. 1) to the grippers 426, 428 through various structural members and power transmitting members which include the connector 16b, the distal joint 47, the gear bodies 130, 308, 406, 408, the main shaft assembly 306, the wires 52, 54, 56, the gear rings 152, 320, the first end effector member 418, the second end effector member 420, the shaft 110, and the securing pin 330. The grippers 426, 428 are thus heated to treat the living tissue 400 of the human body. As the grippers 426, 428 can be angularly moved, rotated, and opened and closed freely in the yawing and pitching directions, as described above, the grippers 426, 428 as they grip a desired tool or the like can easily be manipulated to supply the electric current to a desired region of the human body to treat the region.

The manipulator 10d has two insulating plates 319 as with the manipulator 10c. The insulating plates 319 prevent the living tissue 400 from contacting the gear bodies 130, 308, the main shaft assembly 306, and the wires 52, 54, 56 in the joint with the first rotational axis Oy at its center. In particular, even when the end effector 404 is largely bent in a yawing direction, e.g., substantially through a right angle, about the first rotational axis Oy, the two insulating plates 319 turn in unison with the main shaft assembly 306 to close the opening of the joint. Therefore, while the end effector 404 can be moved in a sufficient range and can be cleaned effectively, the insulating plates 319 are continuously capable of preventing the gear body 130, etc. and the living tissue 400 from contacting each other.

The manipulators 10a through 10d and the working units 12a through 12d have been illustrated as being used in the medical application wherein objects to be operated on and environments in which they operate are living bodies. However, they are not limited to the medical application, but are applicable to uses wherein the manipulators 10a through 10d, objects to be operated on, and environments in which they operate need to be insulated.

It can easily be understood that the end effectors 104, 204 of the working units 12a, 12b may be replaced with the grippers 426, 428 of the working unit 12d, and the grippers 426, 428 may be changed in shape and structure into any of various tools including a pliers, a nipper, an end nipper, etc.

In each of the embodiments, the combinations of spur gears and face gears may be replaced with combinations of other elements, e.g., bevel gears, insofar as they can transmit the rotational power through their mutual contact while changing the rotational direction.

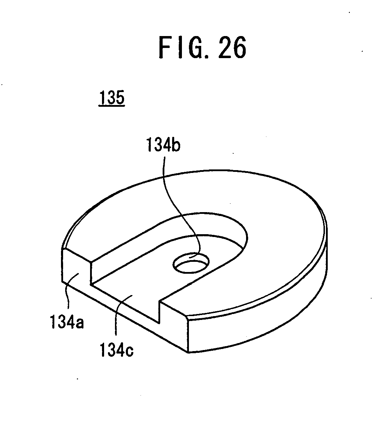

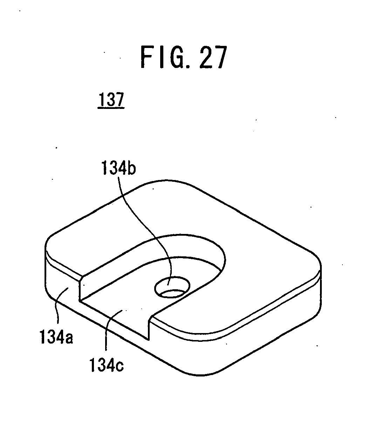

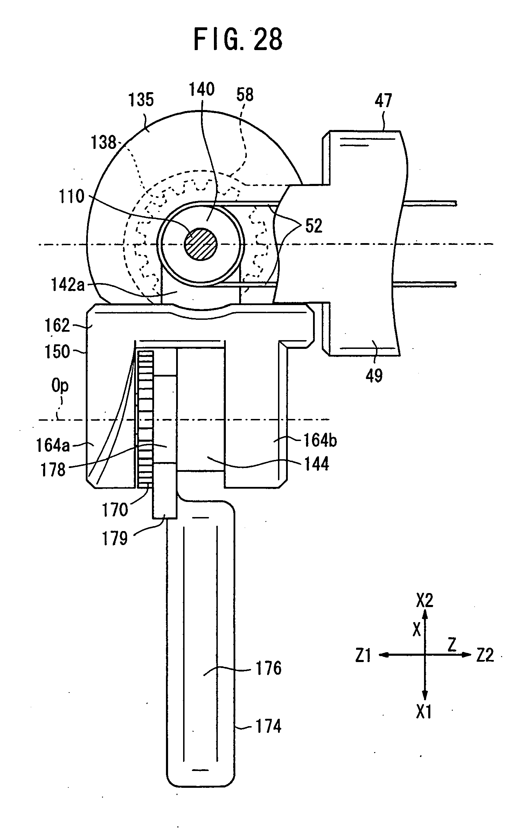

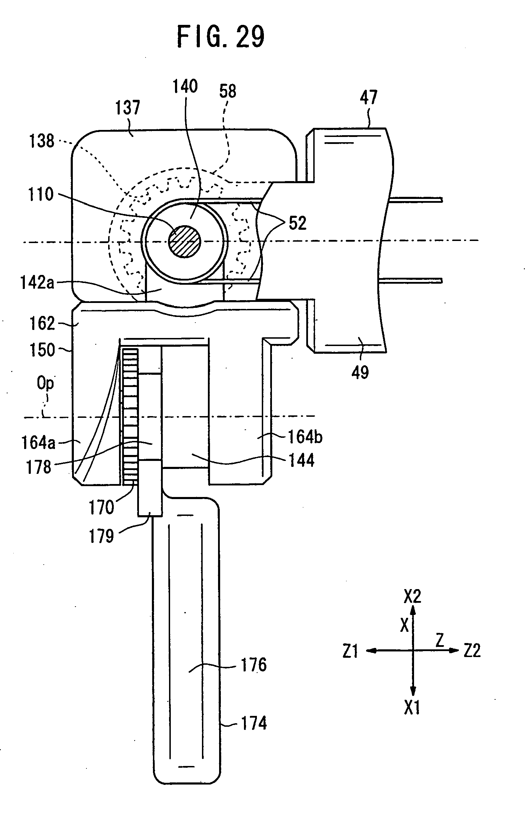

The insulating plates 134, 319 may be of various shapes such as a shape with dual arcuate edges and a rectangular shape insofar as they are capable of preventing the object to be operated on (e.g., the living tissue 400, a medical instrument, a suture, etc.) and the power transmitting members (e.g., the gear 138, etc.) from contacting each other. For example, the insulating plate 134 may be replaced with a substantially oval-shaped insulating plate 135 (see FIG. 26) or a rectangular insulating plate 137 with round corners (see FIG. 27). If the insulating plate 135 is incorporated in the working unit 12a, then the amount of outward projection (radius) thereof increases as the angle through which the working unit 12a is angularly moved in a yawing direction increases (see FIG. 28). If the insulating plate 137 is incorporated in the working unit 12a, then when the working unit 12a is angularly moved through a substantially right angle in a yawing direction, the insulating plate 137 has a diagonal direction tilted at 45° with a maximum amount of outward projection (radius) (see FIG. 29).

A manipulator (10a) includes a working unit (12a) comprising an operation command unit (14a), horizontal roller and vertical rollers (34, 35) mounted thereon, drive pulleys (50a, 50b) rotatable in response to operation of the horizontal roller (35) and the vertical roller (34), a connector (16a), a first rotational axis (Oy) disposed on a distal end of the connector (16a), a second rotational axis (Op) extending perpendicularly to the first rotational axis (Oy), tubular members (140, 136) rotatably supported on a shaft providing the first rotational axis (Oy), and wires (52, 54) having rear and front portions trained around the drive pulleys (50a, 50b) and the tubular members (140, 136), respectively. A drive mechanism (102) operates about the first rotational axis (Oy) in response to rotation of the tubular member (140), and an end effector (104) operates about the second rotational axis (Op) in response to rotation of the tubular member (136).

## Claims

1. A manipulator comprising:
an operating unit (14a, 14b);
a first input unit (35) and a second input unit (34) which are included in said operating unit (14a, 14b);
a first rotational source (50a) and a second rotational source (50b) which are configured to rotate in response to operation of said first input unit (35) and said second input unit (34);
a connector (16a, 16b) having a proximal end connected to said operating unit (14a, 14b) or a drive unit for rotating said first rotational source (50a) and said second rotational source (50b);
a first joint shaft (110) disposed on a distal end of said connector (16a, 16b);
a second joint shaft (156, 208) extending across said first joint shaft (110);
a first tubular member (140) and a second tubular member (136) which are rotatably supported about said first joint shaft (110);
a drive mechanism (102, 202, 302, 402) configured to operate about said first joint shaft (110) in response to rotation of said first tubular member (140); and
an acting unit (104, 204, 304, 404) disposed ahead of said drive mechanism (102, 202, 302, 402) and configured to operate about said second joint shaft (156, 208) in response to rotation of said second tubular member (136);
**characterized by**
a first flexible power transmitting member (52) for transmitting electric and/or mechanic power having a rear portion trained around said first rotational source (50a) and a front portion trained around said first tubular member (140);
a second flexible power transmitting member (54) for transmitting electric and/or mechanic power having a rear portion trained around said second rotational source (50b) and a front portion trained around said second tubular member (136);
a first electrically insulating member (134, 319) rotatably supported about said first joint shaft (110),
wherein said first electrically insulating member (134, 319) comprises at least a partly circular plate having the outer radius which is equal to or greater than the sum of the outer radius of said first tubular member (140) and the diameter of said first flexible power transmitting member (52), and equal to or greater than the sum of the outer radius of said second tubular member (136) and the diameter of said second flexible power transmitting member (54).

2. A manipulator according to claim 1, wherein said first electrically insulating member (134, 319) is disposed between said first tubular member (140) and said second tubular member (136).

3. A manipulator according to claim 1, further comprising:
a third input unit (32) included in said operating unit (14a, 14b);
a third rotational source (50c) which is configured to rotate in response to operation of said third input unit (32);
a third joint shaft (352) extending perpendicularly to or coaxially with said second joint shaft (156, 208);
a third tubular member (310) rotatably supported about said first joint shaft (110);
a third flexible power transmitting member (56) for transmitting electric and mechanic power having a rear portion trained around said third rotational source (50c) and a front portion trained around said third tubular member (310); and
a second electrically insulating member (319) rotatably supported about said first joint shaft (110);
wherein said acting unit (104, 204, 304, 404) is configured to operate about said third joint shaft (352) in response to rotation of said third tubular member (310).

4. A manipulator according to claim 3, wherein said first tubular member (140) is disposed between said second tubular member (136) and said third tubular member (310), said first electrically insulating member (134, 319) is disposed between said first tubular member (140) and said second tubular member (136), and said second electrically insulating member (319) is disposed between said first tubular member (140) and said third tubular member (310).

5. A manipulator according to claim 3, wherein said partly circular plate has a radius which is equal to or greater than the sum of the outer radius of said third tubular member (310) and the diameter of said third flexible power transmitting member (56).

6. A manipulator according to claim 3, wherein said first electrically insulating member (134, 319) and said second electrically insulating member (319) are configured to rotate in response to rotation of said first tubular member (140).

## Patentansprüche

1. Manipulator mit:
einer Betätigungseinheit (14a, 14b);
einer ersten Eingabeeinheit (35) und einer zweiten Eingabeeinheit (34), die in der Betätigungseinheit (14a, 14b) enthalten sind;
einer ersten Rotationsquelle (50a) und einer zweiten Rotationsquelle (50b), die aufgebaut sind, um sich in Reaktion auf eine Betätigung der ersten Eingabeeinheit (35) und der zweiten Eingabeeinheit (34) zu drehen;
einer Verbindungseinrichtung (16a, 16b), die ein proximales Ende aufweist, das mit der Betätigungseinheit (14a, 14b) oder einer Antriebseinheit zum Drehen der ersten Rotationsquelle (50a) und der zweiten Rotationsquelle (50b) verbunden ist;
einer ersten Gelenkwelle (110), die an einem distalen Ende der Verbindungseinrichtung (16a, 16b) angeordnet ist;
einer zweiten Gelenkwelle (156, 208), die sich quer zu der ersten Gelenkwelle (110) erstreckt;
einem ersten röhrenartigen Bauteil (140) und einem zweiten röhrenartigen Bauteil (136), die drehbar um die erste Gelenkwelle (110) gestützt sind;
einem Antriebsmechanismus (102, 202, 302, 402), der aufgebaut ist, um in Reaktion auf eine Drehung des ersten röhrenartigen Bauteils (140) um die erste Gelenkwelle (110) herum betätigt zu werden; und
einer Arbeitseinheit (104, 204, 304, 404), die vor dem Antriebsmechanismus (102, 202, 302, 402) angeordnet ist und aufgebaut ist, um in Reaktion auf eine Drehung des zweiten röhrenartigen Bauteils (136) um die zweite Gelenkwelle (156, 208) herum betätigt zu werden; **gekennzeichnet, durch**
ein erstes flexibles Leistungsübertragungsbauteil (52) zum Übertragen von elektrischer und/oder mechanischer Leistung, das einen hinteren Abschnitt aufweist, der um die erste Rotationsquelle (50a) herum verläuft, und einen vorderen Abschnitt aufweist, der um das erste röhrenartige Bauteil (140) herum verläuft;
ein zweites flexibles Leistungsübertragungsbauteil (54) zum Übertragen von elektrischer und/oder mechanischer Leistung, das einen hinteren Abschnitt aufweist, der um die zweite Rotationsquelle (50b) herum verläuft, und einen vorderen Abschnitt aufweist, der um das zweite röhrenartige Bauteil (136) herum verläuft;
ein erstes elektrisch isolierendes Bauteil (134, 319), das um die erste Gelenkwelle (110) herum drehbar gestützt ist,
wobei das erste elektrisch isolierende Bauteil (134, 319) zumindest eine teilweise kreisförmige Platte aufweist, die einen Außenradius aufweist, der gleich wie oder größer als die Summe von dem Außenradius von dem ersten röhrenartigen Bauteil (140) und dem Durchmesser des ersten flexiblen Leistungsübertragungsbauteils (52) ist und gleich wie oder größer als die Summe von dem Außenradius von dem zweiten röhrenartigen Bauteil (136) und dem Durchmesser des zweiten flexiblen Leistungsübertragungsbauteils (54) ist.

2. Manipulator gemäß Anspruch 1, wobei das erste elektrisch isolierende Bauteil (134, 319) zwischen dem ersten röhrenartigen Bauteil (140) und dem zweiten röhrenartigen Bauteil (136) angeordnet ist.

3. Manipulator gemäß Anspruch 1, der ferner Folgendes aufweist:
eine dritte Eingabeeinheit (32), die in der Betätigungseinheit (14a, 14b) enthalten ist;
eine dritte Rotationsquelle (50c), die aufgebaut ist, um sich in Reaktion auf eine Betätigung der dritten Eingabeeinheit (32) zu drehen;
eine dritte Gelenkwelle (352), die sich senkrecht zu oder koaxial mit der zweiten Gelenkwelle (156, 208) erstreckt;
ein drittes röhrenartiges Bauteil (310), das um die erste Gelenkwelle (110) herum drehbar gestützt ist;
ein drittes flexibles Leistungsübertragungsbauteil (56) zum Übertragen von elektrischer und mechanischer Leistung, das einen hinteren Abschnitt aufweist, der um die dritte Rotationsquelle (50c) herum verläuft, und einen vorderen Abschnitt aufweist, der um das dritte röhrenartige Bauteil (310) herum verläuft; und
ein zweites elektrisch isolierendes Bauteil (319), das um die erste Gelenkwelle (110) herum drehbar gestützt ist;
wobei die Arbeitseinheit (104, 204, 304, 404) aufgebaut ist, um in Reaktion auf eine Drehung des dritten röhrenartigen Bauteils (310) um die dritte Gelenkwelle (352) herum betätigt zu werden.

4. Manipulator gemäß Anspruch 3, wobei das erste röhrenartige Bauteil (140) zwischen dem zweiten röhrenartigen Bauteil (136) und dem dritten röhrenartigen Bauteil (310) angeordnet ist, wobei das erste elektrisch isolierende Bauteil (134, 319) zwischen dem ersten röhrenartigen Bauteil (140) und dem zweiten röhrenartigen Bauteil (136) angeordnet ist und das zweite elektrisch isolierende Bauteil (319) zwischen dem ersten röhrenartigen Bauteil (140) und dem dritten röhrenartigen Bauteil (310) angeordnet ist.

5. Manipulator gemäß Anspruch 3, wobei die teilweise kreisförmige Platte einen Radius hat, der gleich wie oder größer als die Summe von dem Außenradius von dem dritten röhrenartigen Bauteil (310) und dem Durchmesser von dem dritten flexiblen Leistungsübertragungsbauteil (56) ist.

6. Manipulator gemäß Anspruch 3, wobei das erste elektrisch isolierende Bauteil (134, 319) und das zweite elektrisch isolierende Bauteil (319) aufgebaut sind, um sich in Reaktion auf eine Drehung des ersten röhrenartigen Bauteils (140) zu drehen.

## Revendications

1. Manipulateur comprenant:
une unité opérationnelle (14a, 14b);
une première unité d'entrée (35) et une deuxième unité d'entrée (34) qui sont comprises dans ladite unité opérationnelle (14a, 14b);
une première source de rotation (50a) et une deuxième source de rotation (50b) qui sont configurées pour se mettre en rotation en réponse à un fonctionnement de ladite première unité d'entrée (35) et de ladite deuxième unité d'entrée (34);
un connecteur (16a, 16b) ayant une extrémité proximale reliée à ladite unité opérationnelle (14a, 14b) ou une unité d'entraînement pour mettre en rotation ladite première source de rotation (50a) et ladite deuxième source de rotation (50b);
un premier arbre à joint (110) disposé sur une extrémité distale dudit connecteur (16a, 16b);
un deuxième arbre à joint (156, 208) s'étendant à travers ledit premier arbre à joint (110);
un premier élément tubulaire (140) et un deuxième élément tubulaire (136) qui sont soutenus en rotation autour dudit premier arbre à joint (110);
un mécanisme d'entraînement (102, 202, 302, 402) configuré pour fonctionner autour dudit premier arbre à joint (110) en réponse à une rotation dudit premier élément tubulaire (140); et
une unité d'action (104, 204, 304, 404) disposée en avant dudit mécanisme d'entraînement (102, 202, 302, 402) et configurée pour fonctionner autour dudit deuxième arbre à joint (156, 208) en réponse à la rotation dudit deuxième élément tubulaire (136);
**caractérisé par**
un premier élément flexible (52) de transmission de puissance pour transmettre de la puissance électrique et/ou mécanique ayant une partie arrière entraînée autour de ladite première source de rotation (50a) et une partie avant entraînée autour dudit premier élément tubulaire (140);
un deuxième élément flexible (54) de transmission de puissance pour transmettre la puissance électrique et/ou mécanique ayant une partie arrière entraînée autour de ladite deuxième source de rotation (50b) et une partie avant entraînée autour dudit deuxième élément tubulaire (136);
un premier élément (134, 319) électriquement isolant soutenu en rotation autour dudit premier arbre à joint (110),
où ledit premier élément (134, 319) électriquement isolant comprend au moins une plaque partiellement circulaire dont le rayon externe est supérieur ou égal à la somme du rayon externe dudit premier élément tubulaire (140) et du diamètre dudit premier élément flexible (52) de transmission de puissance, et supérieur ou égal à la somme du rayon externe dudit deuxième élément tubulaire (136) et du diamètre dudit deuxième élément flexible (54) de transmission de puissance.

2. Manipulateur selon la revendication 1, dans lequel ledit premier élément (134, 319) électriquement isolant est disposé entre ledit premier élément tubulaire (140) et ledit deuxième élément tubulaire (136).

3. Manipulateur selon la revendication 1, comprenant en plus:
une troisième unité d'entrée (32) comprise dans ladite unité opérationnelle (14a, 14b);
une troisième source de rotation (50c) qui est configurée pour se mettre en rotation en réponse à un fonctionnement de ladite troisième unité d'entrée (32);
un troisième arbre à joint (352) s'étendant de manière perpendiculaire à ou coaxiale avec ledit deuxième arbre à joint (156, 208);
un troisième élément tubulaire (310) soutenu en rotation autour dudit premier arbre à joint (110);
un troisième élément flexible (56) de transmission de puissance pour transmettre de la puissance électrique et mécanique ayant une partie arrière entraînée autour de ladite troisième source de rotation (50c) et une partie avant entraînée autour dudit troisième élément tubulaire (310); et
un deuxième élément (319) électriquement isolant soutenu en rotation autour dudit premier arbre à joint (110);
où ladite unité d'action (104, 204, 304, 404) est configurée pour fonctionner autour dudit troisième arbre à joint (352) en réponse à une rotation dudit troisième élément tubulaire (310).

4. Manipulateur selon la revendication 3, dans lequel ledit premier élément tubulaire (140) est disposé entre ledit deuxième élément tubulaire (136) et ledit troisième élément tubulaire (310), ledit premier élément (134, 319) électriquement isolant est disposé entre ledit premier élément tubulaire (140) et ledit deuxième élément tubulaire (136), et ledit deuxième élément (319) électriquement isolant est disposé entre ledit premier élément tubulaire (140) et ledit troisième élément tubulaire (310).

5. Manipulateur selon la revendication 3, dans lequel ladite plaque partiellement circulaire a un rayon qui est supérieur ou égal à la somme du rayon externe dudit troisième élément tubulaire (310) et du diamètre dudit troisième élément flexible (56) de transmission de puissance.

6. Manipulateur selon la revendication 3, dans lequel ledit premier élément (134, 319) électriquement isolant et ledit deuxième élément (319) électriquement isolant sont configurés pour se mettre en rotation en réponse à une rotation dudit premier élément tubulaire (140).
